# EUROPEAN PATENT APPLICATION

(11) **EP 2 692 731 A1**
(43) Date of publication of application: **05.02.2014**
(21) Application number: 12005565.2
(22) Date of filing: 31.07.2012
(51) Int. Cl.: C07K 14/005, A61K 48/00, C12N 15/86

(54) **Recombinant Adeno-Associated virus (AAV) vector particles displaying high-affinity ligands for cell-type specific gene delivery**

(71) Applicant: Paul-Ehrlich-Institut Bundesamt für Sera und Impfstoffe, 63225 Langen (DE); Universität zu Köln, 50923 Köln (DE)
(72) Inventor: Bucholz, Christian, 60594 Frankfurt (DE); Münch, Robert, 60596 Frankfurt (DE); Büning, Hildegard, 50933 Köln (DE)
(74) Representative: Schüssler, Andrea

(57) **Abstract**

The present invention relates to recombinant adeno-associated virus (AAV) vector particles displaying high-affinity ligands, which can be used for cell-type specific gene delivery during therapeutic applications and applications in basic research, since they provide high cell-type selectivity or rather high targeting specificity, and to methods for their construction and generation.

In detail, the present invention refers to a recombinant AAV vector particle, wherein the differences to the AAV wild-type particle are based on (a) at least one packaged transgene to be delivered to a specific target cell or target tissue, (b) a mutated AAV capsid protein VP2, wherein the mutation is based on a cell-type specific DARPin that is fused to the N-terminus of the capsid protein VP2, (c) a mutated VP2-start codon, and (d) a capsid, wherein the essential binding site for the natural receptor of the capsid proteins VP1, VP2 and VP3 is mutated.

## Description

### Technical Field

The present invention relates to recombinant Adeno-Associated Virus (AAV) vector particles displaying high-affinity ligands for cell-type specific gene delivery. More specifically, the present invention provides recombinant AAV vector particles and methods for their construction and generation, which display high-affinity ligands exemplified for cell-type specific designed ankyrin repeat proteins (DARPins) that specifically transduce target cells or target tissues, which express the respective target receptor, such as HER2/*neu*-positive or CD4-positive cells.

### Background of the invention

Virus-derived vectors have become one of the most promising gene delivery systems for mammalian cells. Especially Adeno-Associated Virus (AAV) vector particles provide an outstanding potential as gene transfer vectors and are thought to be best suited for *in vivo* gene delivery from the portfolio of gene transfer vectors (Gao, G. et al., J. Virol., 2004, 6381-6388). The established features of AAV vector particles that distinguish this viral vector from other vectors include stable long-term expression, broad host range, ability to transduce proliferating and post-mitotic cells, high titers of AAV vectors produced in tissue cultures, derivation from a nonpathogenic virus and low immunogenicity of both wild type virus and vectors (Kotin, R. M., Hum. Gene Ther., 1994, 793-801).

The AAV is a member of the genus Dependovirus, which lies in the *Parvoviridae* (parvovirus family). An interest in this family of viruses has been stimulated because of their potential use as gene transfer vectors (Gao, G. et al., J. Virol., 2004, 6381-6388). From the portfolio of all different types of gene transfer vectors, AAV vectors are thought to be best suited for *in vivo* gene delivery. Due to their substantially smaller size as compared to lentiviral vectors, they show much higher diffusion rates in tissue and can therefore, in principle, transduce large areas of tissue upon local injection. Moreover, they do not integrate the vector genome into the cellular genome and, therefore, are not prone to insertional oncogenesis as observed with retro- and lentiviral vectors in clinical trials. Despite their success as gene transfer vector system, however, vector design problems remain. One major concern is the fact that following systemic application in mice or non-human primates, AAV vector particles tend to accumulate in the liver, which limits efficient transduction of other target tissues (Gao, G. et al., J. Virol., 2004, 6381-6388). Furthermore, systemic overexpression of the transgene, e.g. cytokines, from liver or other tissue, which were transduced by recombinant AAV vector particles (rAAV) owning to its broad tropism, could have toxic effects. As further consequence of the broad tropism, transduction efficacy of target organs is low and hence high vector doses need to be applied. Furthermore, if the transgene product is toxic by itself or can modify a prodrug to toxic metabolite, transduction of non-target cells, which is a result of the promiscuous tropism, raises safety concerns as healthy cells become eradicated. Although gene expression may be controlled by tissue-specific promoters, vector particles can still enter non-target cells and induce e.g. cytotoxicity (Mingozzi, F. & K. A. High, Nat. Rev. Genet., 12, 341-355). Consequently, since cell-type specific gene delivery is advantageous, e.g. for delivery of a gene product to a tumor that is localized at a particular anatomical site (but not, e.g. to surrounding non-cancerous tissue), for delivery of a gene product to a diseased tissue (but not, e.g. to the surrounding healthy tissue), and for many more applications including therapeutic applications as well as applications in basic research, cell surface targeting technologies aiming to restrict vector entry and gene delivery to specific cell-types are under development since 1993 (Waehler, R. et al., Nat. Rev. Genet., 2007, 573-587).

Significant improvements of AAV vectors with respect to their specificity occurred mainly by direct or indirect modification of the capsid, which represents the interface to cellular receptors and antibodies and is represented by capsid proteins: VP1, VP2 and VP3, which interact together in a ratio of 1:1:10 to form a capsid of an icosahedral symmetry (Büning, H. et al., J. Gene Med., 2008, 10, 717-733). First efforts, focused on exploitation of alternative serotypes, provided a first opportunity to improve gene transfer efficiencies to tissues, wherein pseudotyping, i.e. cross-packaging of a genome of one serotype into the capsid of another serotype, created infectious vectors with the tropism of the new capsid (Davidson, B. L. et al., Proc. Natl. Acad. Sci. USA, 2000, 97, 3428-3432). However, despite the improvements in tropism achieved by pseudotyping, an increase of specificity to a specific cell-type by this method remains impossible.

Moreover, the patent application US 2006/0286545 A1 provides for a method of selecting a virus, which comprises a variant of a capsid or envelope protein that alters tropism of the virus. This method comprises: (a) infecting host cells with a pseudotyped viral particle from an AAV peptide library in which random peptides are displayed within the viral capsid; (b) contacting target cells with viral particles produced from the infected cells of step (a) at a multiplicity of infection of less than 1; and (c) detecting successful infection of the target cells, wherein successful infection indicates that the tropism of the virus is altered such that it infects the target cell. However, although the results just described demonstrated high transgene expression in specific cell-types, they do not rigorously prove that high infection titers can be achieved.

Furthermore, the authors of US 2009/0202490 A1 developed a recombinant AAV vector comprising mutant capsid proteins, wherein the recombination was based on at least one amino acid substitution relative to the corresponding parental AAV capsid protein. By introducing this mutation, such recombinant AAV vectors exhibited one or more of the following properties which include altered, i.e. increased or decreased heparin binding affinity relative to wild-type AAV and/or altered infectivity of particular cell types, e.g. of cell types that are resistant to infection with AAV. However, although such a patent application is related to problems including limited tissue dispersion for AAV serotypes that employ heparan sulfate as the natural receptor or poor infection of non-permissive cell types such as stem cells, and to challenges with high efficiency targeting of gene delivery to selected cell populations, there is no report on an absolutely specific gene transfer.

Thus, current methodology that depends on the direct or indirect modification of capsid components by inserting or substituting at least one amino acid does not provide an efficient and completely specific transduction of target cells or target tissues. This may be due to the accessibility of cell-type specific receptors during selection procedure using e.g. peptide libraries, the fact that the identity of target receptors is unknown, and the rigid capsid structure so that re-targeting attempts so far relied on small peptides, i.e. on peptides comprising 7-8 amino acids at the maximum. Such small peptides, however, provide a limiting specificity only, which leads to a limiting receptor affinity. Inserted at capsid protrusions, they conferred improved transduction efficacy but only low-cell type selectivity. Ried, M. U. et al. (J. Virol., 2002, 76, 4559-4566) admittedly disclosed the insertion of 34 amino acids plus linker sequences, showing that this is the size that can be maximally inserted.

### Problem of the invention

Thus, the problem of the present invention is to provide recombinant AAV vector particles with the ability to display high-affinity ligands for cell-type specific gene delivery in order to provide high cell-type selectivity or rather high targeting specificity.

### Summary of the invention

The technical problem of the invention is solved by the subject-matter of independent claims 1 and 13, wherein preferred embodiments are the subject-matter of the dependent claims.

The inventors developed recombinant AAV vector particles displaying high-affinity ligands, which can be used for cell-type specific gene delivery providing high cell-type selectivity or rather high targeting specificity allowing restricted biodistribution and safe gene transfer and/or delivery of genes or rather transgenes.

Their generation is mainly based on the modification of the AAV capsid protein VP2, wherein its N-terminus serves as the platform to display a cell-type specific DARPin. The DARPins of the present invention are designed ankyrin repeat proteins, which represent high affinity multi-domain protein ligands with a molecular mass of about 14 kDa. The binding interface is flanked by two capping repeats and comprises 50-200, preferably 100-120 amino acids. They show exceptional expression yields, stabilities and affinities in the nanomolar range that direct recombinant AAV vector particles specifically to the target cell or rather target tissue. Thus, the combination of AAV vector particles with cell-type specific DARPins, which are specific for at least one surface receptor, preferably for one surface receptor, that is expressed on the target cell or target tissue, provides an improved re-targeting system for AAV vector particles that allows cell-type specific gene delivery with high cell-type selectivity or rather high targeting specificity and, thus, a re-targeting system for AAV vector particles that overcomes the problems of the current methodology, which have been described above.

In detail, the present invention refers to a recombinant AAV vector particle, wherein the differences to the AAV wild-type particle are based on the following features:
(a) at least one packaged transgene, comprising a transgene that encodes for a therapeutically active product and/or a transgene that encodes for a marker/reporter protein,
(b) a mutated AAV capsid protein VP2, wherein the mutation is based on a cell-type specific DARPin that is fused to the N-terminus of the AAV capsid protein VP2 ("DARPin-VP2 fusion protein"), wherein a removable His-tag is optionally fused to the N-terminal end of the DARPin-VP2 fusion protein,
(c) a mutated VP2-start codon, preferably by point mutation, and
(d) a capsid, wherein the essential binding site for the natural receptor of the AAV capsid proteins VP1, VP2 and VP3 is mutated.

Following, the methods for the construction and generation of the recombinant AAV vector particles of the present invention are mainly based on a transfection method, which requires the use of:
(i) a vector plasmid comprising the at least one packaged transgene as described in feature (a),
(ii) a plasmid encoding the DARPin-VP2 fusion protein ("pDARPin-VP2"), wherein VP2 comprises the mutation in the VP2-start codon and in the essential binding site as described in features (c) and (d), and wherein a removable His-tag is optionally fused to the N-terminal end of the DARPin-VP2 fusion protein,
(iii) an AAV helper plasmid, preferably "pRCVP2koA", encoding for AAV non-structural proteins, preferably viral Rep proteins (preferably the non-structural Rep proteins Rep78, Rep68, Rep52, Rep40) and AAP (assembly activating protein), and for the capsid proteins VP1, VP2 and VP3, wherein the AAV helper plasmid comprises the mutation in the VP2-start codon and mutations in the essential receptor binding site as described in features (c) and (d),
(iv) at least one adenovirus (Ad) helper plasmid, preferably "pXX6", encoding for genes possessing Ad helper functions, preferably E2A, E4 and VA RNA genes.

### Brief description of the figures

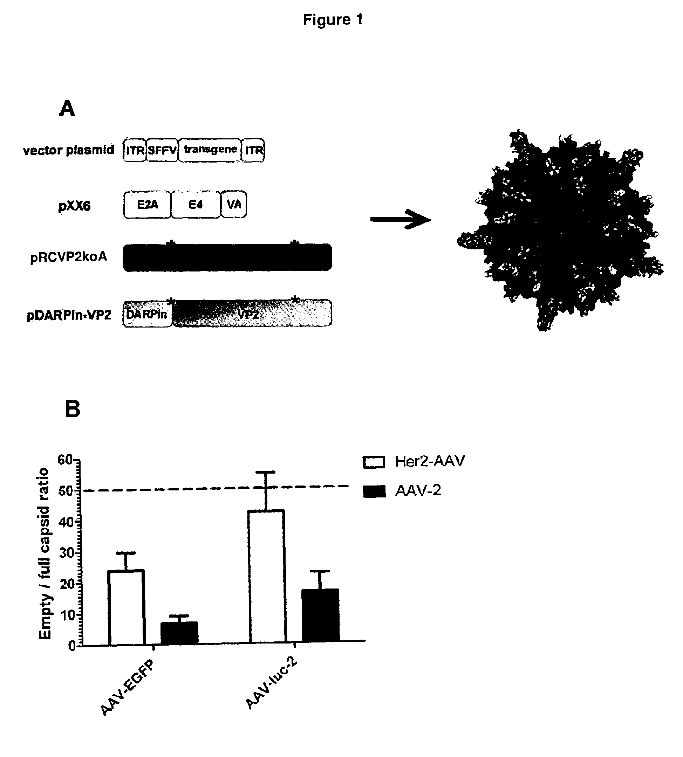

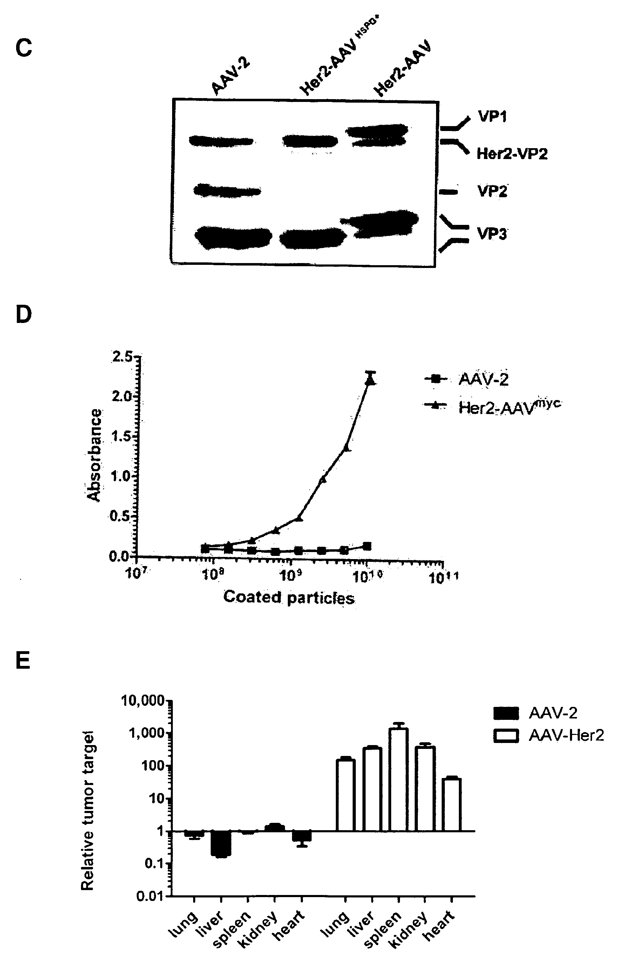

**Figure 1** shows the incorporation and surface display of DARPin on recombinant AAV particles depleted for natural receptor binding.

Figure 1A shows the plasmids used for generation of the recombinant AAV particles showing the open reading frames for capsid proteins (cap ORF) only. In pRCVP2koA and pDARPin-VP2, the VP2-start codon is mutated (labeled by asterisk) to prevent expression of unmodified VP2. To abolish HSPG binding, R585 and R588 were substituted by alanine in pDARPin-VP2 and pRCVP2koA (labeled by asterisk). The Ad helper plasmid pXX6 includes a number of Ad genes, more specifically E2a, E4, and VA RNA genes that possess helper functions, wherein the E4 gene, particularly open reading frame 6, is involved in facilitating AAV DNA replication, and E2a and VA RNA act to enhance the viral mRNA stability and efficiency of translation (Xiao, X. et al., J. Virol., 1998, 72, 2224-2232). Reporter and suicide genes are under control of the SSFV promoter. right: Schematic illustration of the up to five DARPin molecules (red) extruding from pores at the five-fold symmetry axis of the AAV capsid. Illustration was created using Pymol and PDB files 1LP3 (Xie, Q. et al., Proc. Natl. Acad. Sci. USA, 2002, 99, 10405-10410) and 2XEE (Kramer, M. A. et al., J. Mol. Biol., 2010, 404, 381-391).

Figure 1B shows that the incorporation of DARPin does not interfere with the packaging efficiency. AAV particles encoding either EGFP (enhanced green fluorescent protein) or luc-2 (luciferase) were analyzed by qPCR and ELISA to determine the genomic particle and the capsid titers, respectively. Packaging efficiency (genomic-to-capsid ratio) ≤ 50 indicates a wild-type phenotype (dotted line; Kern, A. et al., J. Virol., 2003, 77, 11072-11081). Genomic particle titers were determined by qPCR using the transgene specific primers:
GFP-for 5'-GCTACCCCGACCACATGAAG-3',
GFP-rev 5'-GTCCATGCCGAGAGTGATCC-3',
luc-for 5'-TTCGGCTGGCAGAAGCTATG-3',
luc-rev 5'-GCTCGCGCTCGTTGTAGATG-3',
HSV-TK-for 5'-GCAGCAAGAAGCCACGGAAG-3',
HSV-TK-rev 5'-CCAGCAGTTGCGTGGTGGTG-3'.

Capsid titers were determined by ELISA using the capsid-specific antibody A20 (Wobus, C. E. et al., J. Virol., 2000, 74, 9281-9293). Data represent means of three independent measurements with SD (standard deviation).

Figure 1C shows the Western Blot analysis of iodixanol purified AAV particles. In accordance to previous observations, the R585A and R588A mutations lead to a reduced mobility of AAV capsid proteins in SDS page (Boucas, J. et al., J. Gene Med., 2009, 11, 1103-1113).

Figure 1D shows the surface display of DARPin assayed by ELISA. Her2-AAV particles containing a myc-tag fused to the DARPin-VP2 (Her2-AAV^{myc}) were bound to ELISA-plates coated with a myc-tag specific antibody. Bound vector particles were quantified using the AAV-2 capsid specific antibody A20; N=3 experiments; mean ± SD.

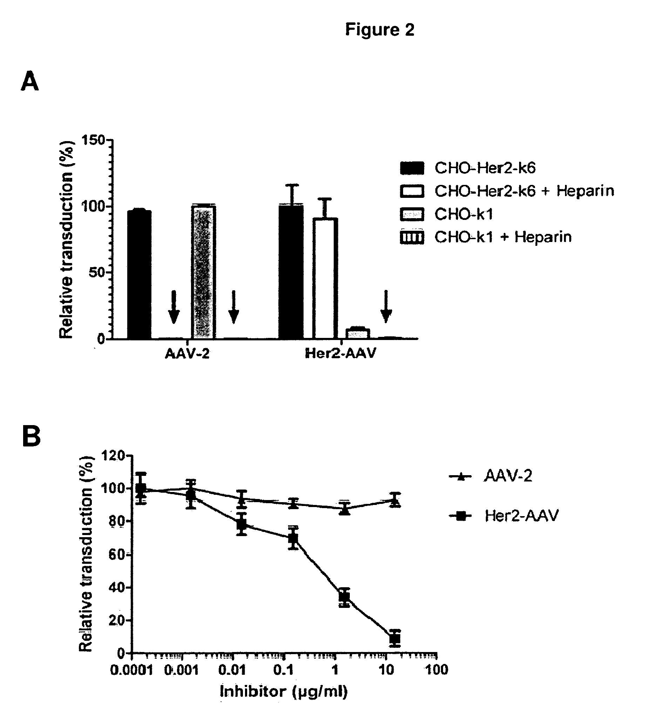

Figure 1E illustrates the tumor targeting using Her2-AAV and AAV-2 particles. Tumor tissue and the indicated organs were explanted from Her2-AAV and AAV-2-injected mice shown in Fig. 2a. Both vector particles encoded for luciferase. Immediately after isolation, samples were lysed and homogenized. Luciferase activities were quantified and normalized to protein content. The relative tumor targeting was calculated as ratio of the luciferase activity in tumor and the luciferase activity in the indicated organ. N=4; mean ± s.e.m. (standard error of the mean).

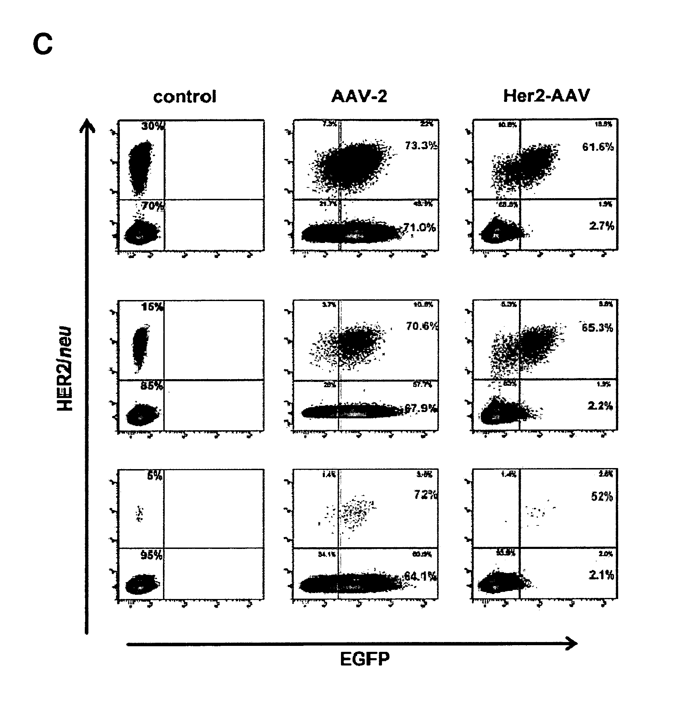

**Figure 2** shows the specificity of gene transfer *in vitro.*

Figure 2A shows CHO-K1 or CHO-Her2-k6 cells, which were incubated with 60,000 genomic particles per cell (GOI) of AAV-2 and Her2-AAV particles encoding for EGFP, respectively, in the absence or presence of heparin (425 IU/ml). The percentage of EGFP-positive cells was quantified by flow cytometry 60 hours post transduction. Values are expressed relative to the highest transduction efficiency for each vector. N=3 experiments; mean ± SD.

Figure 2B shows AAV-2 particles (GOI=3,000) or Her2-AAV particles (GOI=90,000), which were incubated for one hour at 4°C with increasing amounts of the entire HER2/*neu* receptor extracellular domain. Following incubation, SK-OV-3 cells were transduced and analyzed for EGFP expression. Data are normalized to the transduction efficiency measured without pre-incubation with HER2/*neu* domain. N=3 experiments; mean +/- SD.

Figure 2C shows that Her2-AAV particles deliver transgenes specifically to HER2/*neu* positive cells. Therefore, CHO-Her2-k6 and CHO-K1 cells were mixed in different ratios and transduced with Her2-AAV particles (GOI=300,000) or AAV-2 particles (GOI=10,000) or left untreated (control). After 60 hours, the cells were analyzed as described previously (Münch, R. C. et al., Mol. Ther., 2011, 19, 686-693). Percentage of all measured cells for each gate is indicated in black. Values in light grey and dark grey express the percentage of transduced, HER2/*neu*-positive or -negative cells.

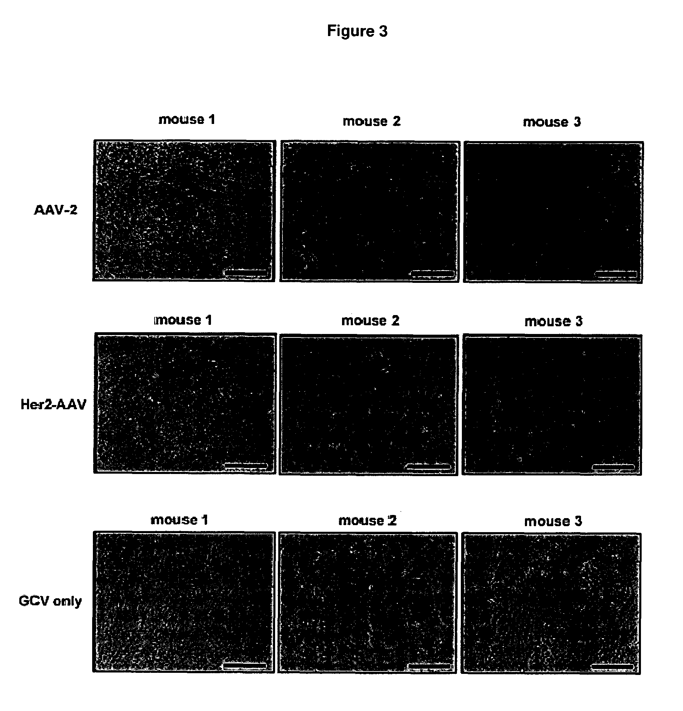

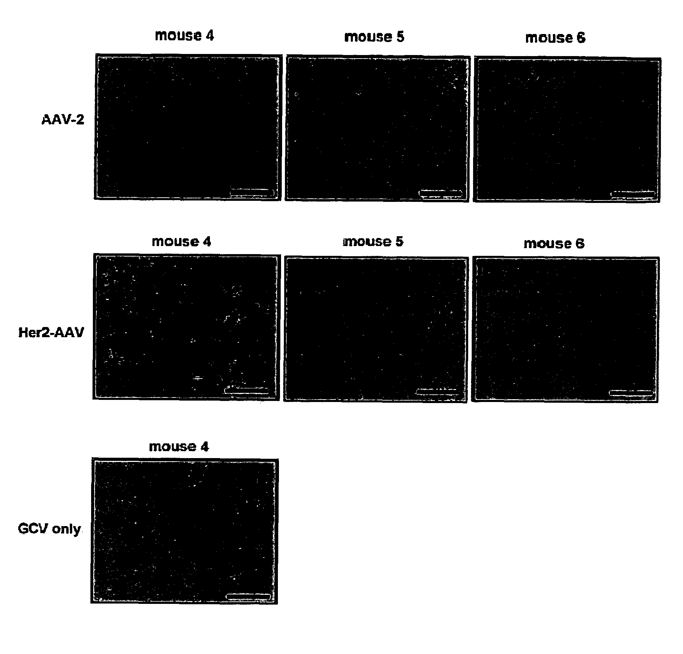

**Figure 3** shows the histological analysis of tumor tissue. Tumor nodules from SK-OV-3 injection sites of mice treated with AAV-2 particles, Her2-AAV particles or GCV only were explanted. Histological analysis of isolated tissues confirmed the presence of SK-OV-3 derived tumors. Representative slices fixed in 4% formalin are shown. Scale bars represent 100 µm.

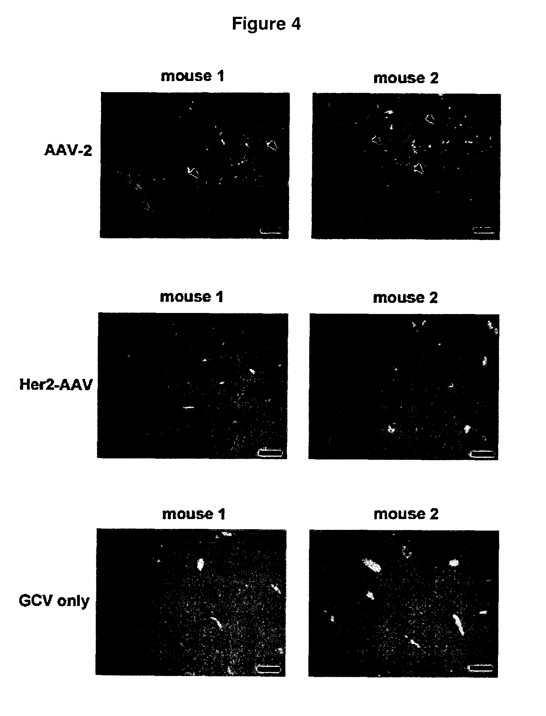

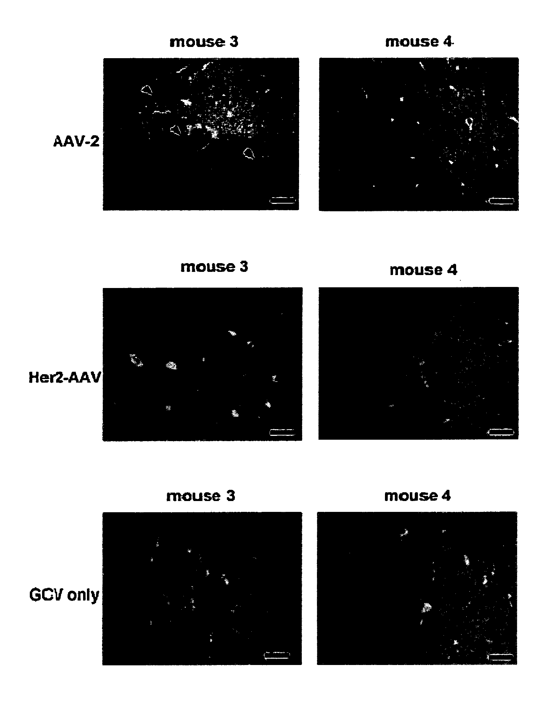

**Figure 4** illustrates that the Her2-AAV particle mediated suicide gene delivery prevents severe liver toxicity. Therefore, SK-OV-3 cell derived tumor bearing mice were intravenously administered with 8x10¹¹ genome copies per animal of AAV-2 particles (N=11), Her2-AAV particles (N=11) or an equal volume of phosphate buffered saline (PBS; N=9) on day 1. GCV-treatment (100 mg/kg body weight) was carried out daily from day 3 to day 9. Representative liver histologies of treated mice are shown. Total liver tissue from mice of each group was fixed in 4% formalin for six days. Fixed tissue was paraffin embedded and 3 µm slices were haematoxylin and eosin stained. Representative sections of four mice from each group are shown. Severe central lobular necrosis (arrow heads) was only detectable in mice of the AAV-2 group that had to be sacrificed due to weight loss before completion of the observation period. Scale bars represent 200 µm.

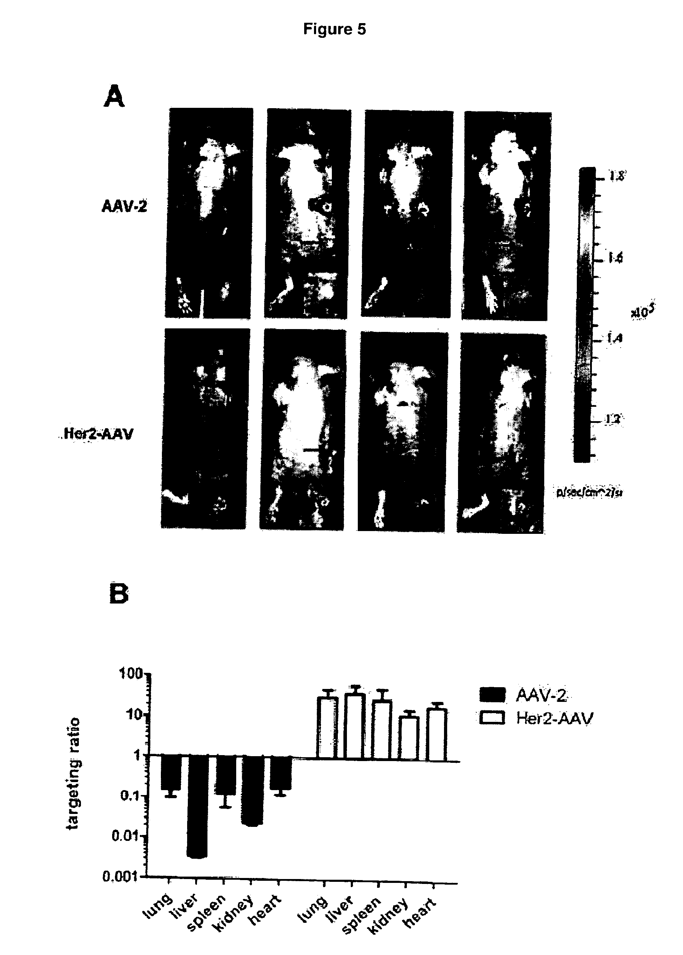

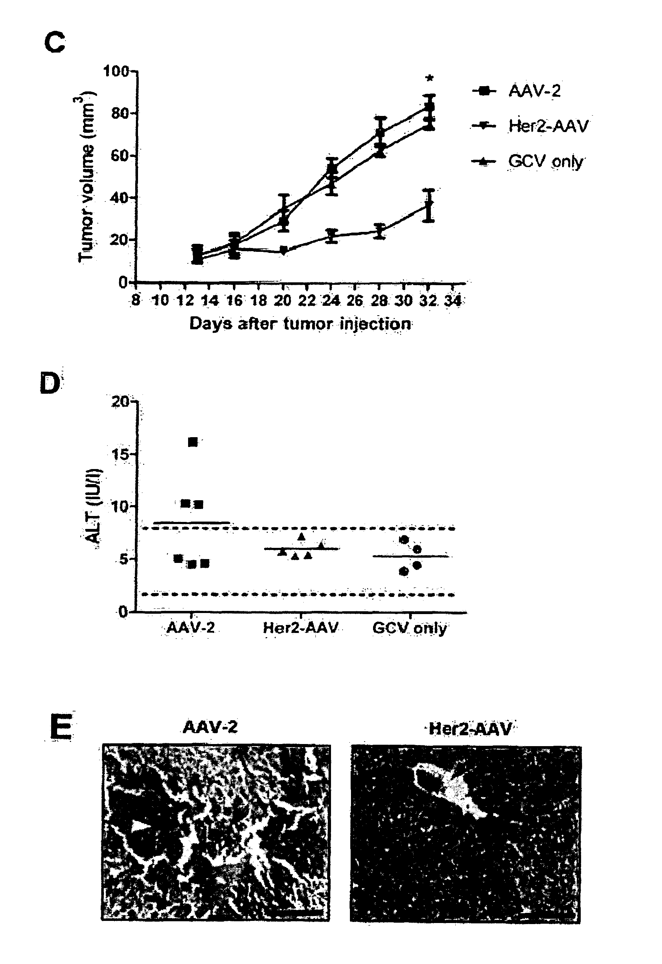

**Figure 5** illustrates that Her2-AAV particles target HER2/*neu*-positive tumors *in vivo* and reduce tumor growth without inducing liver toxicity.

Figure 5A shows the *in vivo* imaging of nude mice carrying subcutaneously growing SK-OV-3 derived tumors (arrow). Data were monitored one week after intravenous injection of AAV-2 particles or Her2-AAV particles (GOI=2x10¹⁰, respectively) transferring the luc-2 expression cassette. Luciferase signal intensity is expressed as photons/second/square centimeter/steradian (p/sec/cm²/sr). Boxes show a magnification of the tumor cell injection site.

Figure 5B shows the biodistribution of AAV particles. Immediately after imaging, mice were sacrificed, indicated organs were isolated and the luciferase gene copies were quantified by qPCR. The targeting coefficient was calculated by normalizing the copy number in tumor tissue to that of the organ indicated. N=4; mean ± s.e.m.

Figure 5C shows the analysis of SK-OV-3 tumor bearing mice, which received a single intravenous injection of HSV-TK transferring AAV-2 particles or Her2-AAV particles (GOI=8x10¹¹, respectively) or PBS, followed by GCV treatment for seven consecutive days. Tumor volume was determined at the indicated time points. AAV-2: N=6, Her2-AAV: N=5, PBS: N=4; mean ± SEM, asterisks indicate p < 0.005 (unpaired t-test).

Figure 5D shows the serum alanine transaminase levels (ALT) of SK-OV-3 cell derived tumor bearing mice, which were intravenously administered with 8x10¹¹ genome copies per animal of AAV-2 (N=11), Her2-AAV (N=11) or an equal volume of PBS (N=9) on day 1. GCV-treatment (100 mg/kg body weight) was carried out daily from day 3 to day 9. In total seven mice of the AAV-2 group had to be sacrificed due to dramatic weight loss. All other mice were sacrificed on day 27. The upper and lower limits of normal are indicated by dotted lines.

Figure 5E shows representative liver histology slices of an AAV-2- and a Her2-AAV-particle treated mouse. Vessels are indicated by black arrows, areas of central lobular necrosis are indicated by black arrow heads and the hypertrophic state with karyorrhexis by white arrow heads. Scale bar represents 100 µm.

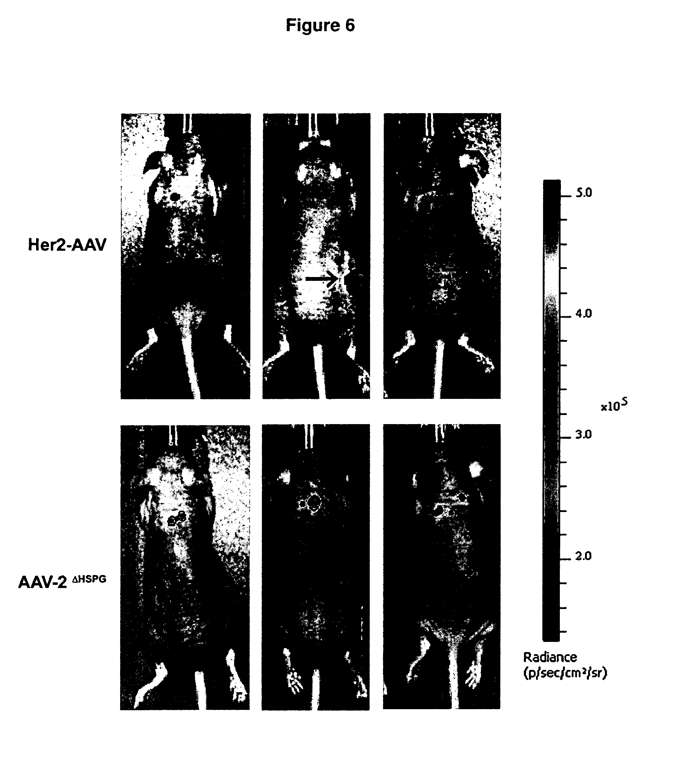

**Figure 6** shows the injection of AAV-2^{ΔHSPG} in comparison to Her2-AAV preparations in SK-OV-3 bearing mice. In AAV-2^{ΔHSPG} treated animals, luciferase signals were confined to the chest region, whereas Her2-AAV mediated signals again were located at the tumor site. Luciferase signal intensity is expressed as photons/second/square centimeter/steradian (p/sec/cm²/sr).

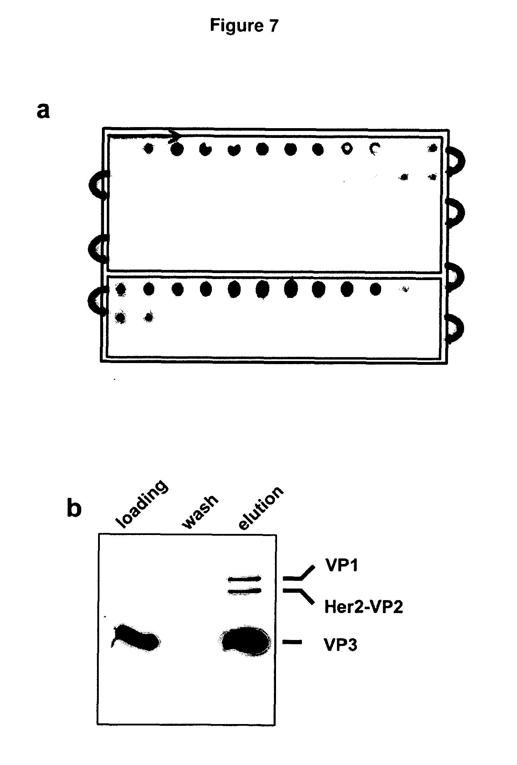

**Figure 7** shows the column purification of Her2-AAV. (a) Iodixanol gradient purified Her2-AAV^{His} preparations were purified using His-Trap-HP-columns (GE Healthcare, Munich, Germany). Step gradient elution (20-500 mM imidazole, 20 mM sodium phosphate and 0.5 M NaCl pH 7.4) was used to elute Her2-AAV^{His} particles. AAV particles in every fraction were identified using dot blot analysis and the AAV capsid protein specific antibody B1. The blue box comprises the loading and the washing fractions; the green box the elution fractions. (b) Loading, wash, and elution fractions were analyzed by Western Blot.

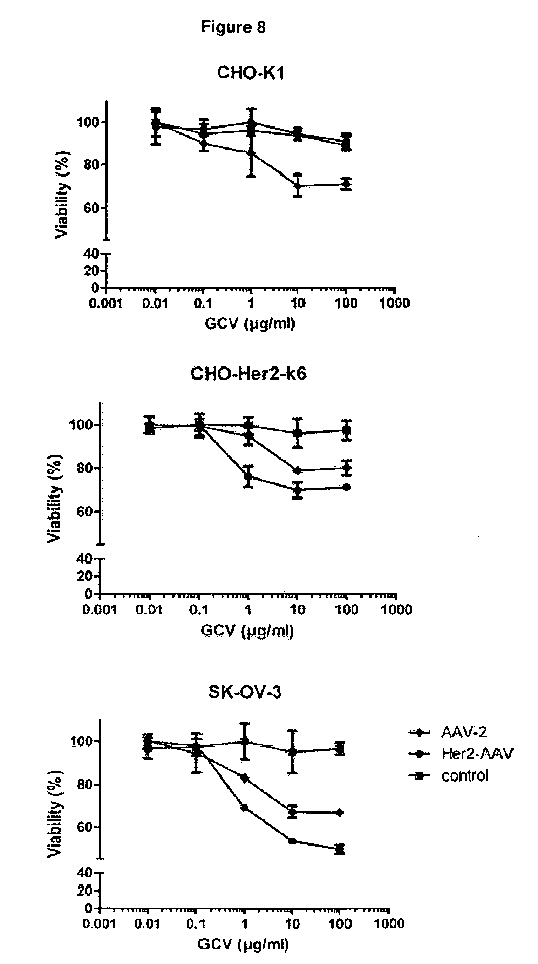

**Figure 8** shows selective HSV-TK delivery to HER2/*neu* positive cell lines. CHO-Her2-k6, SK-OV-3 and CHO-K1 cells were transduced with HSV-TK transferring AAV-2 (GOI=10,000) or Her2-AAV (GOI=300,000) or left untransduced (control). Two days after transduction, cells were incubated with the indicated concentrations of ganciclovir (GCV) for five days. Cell viability was subsequently analyzed using the MTT assay (Roche, Mannheim, Germany). Values were normalized to the cell viability in absence of GCV. Mean values form three independent experiments with SD are shown.

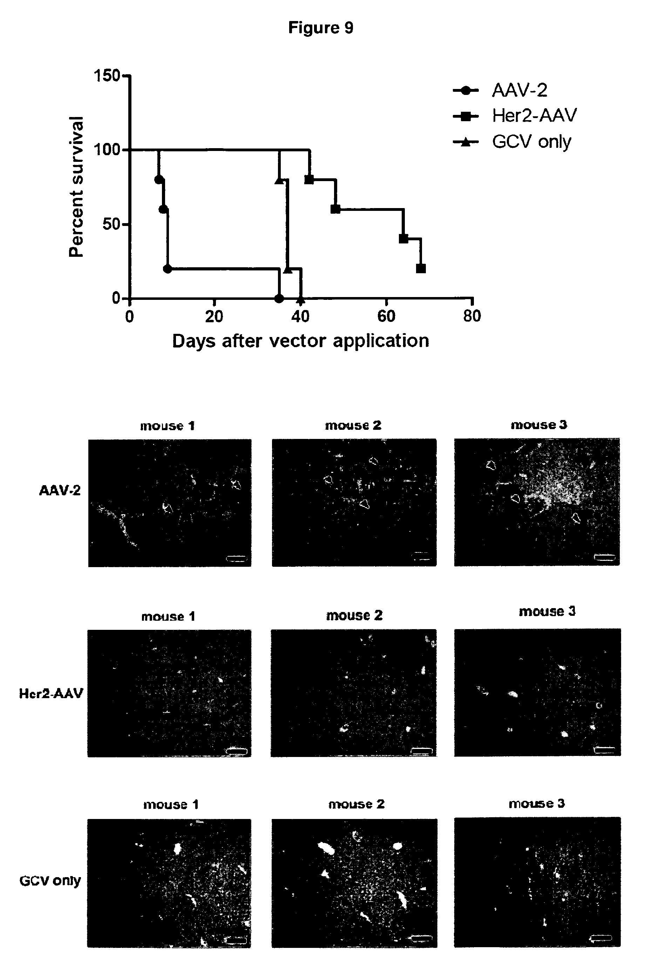

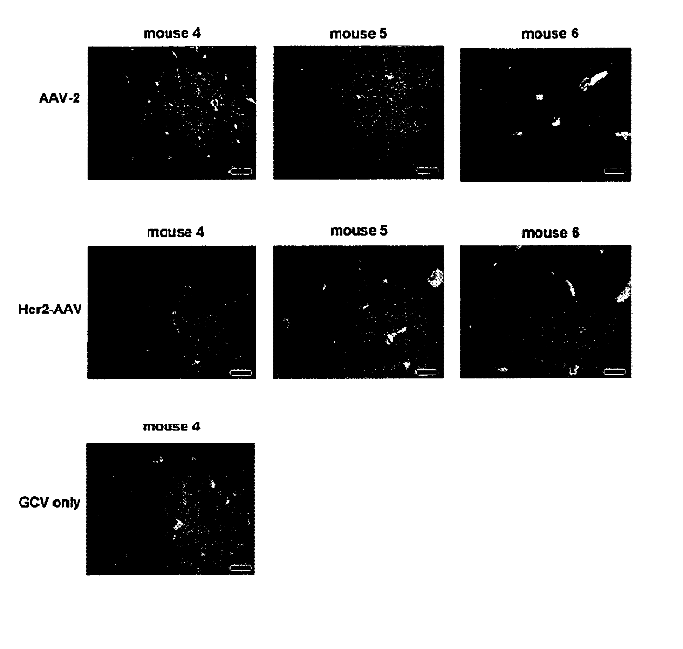

**Figure 9** shows that severe liver toxicity induced by suicide gene approaches can be avoided by cell entry targeting. (A) SK-OV-3 cell derived tumor bearing mice were intravenously administered with 8x10¹¹ genome copies per animal of AAV-2 (N=11), Her2-AAV (N=11) or an equal volume of PBS (N=9) on day 1. GCV-treatment (100 mg/kg body weight) was carried out daily from day 3 to day 9. More than half of the AAV-2 treated mice had to be sacrificed due to dramatic weight loss during the GCV treatment phase. Mock treated animals had to be sacrificed around day 40 due to high tumor burden, (B) AAV-2 treated mice no. 1, 2 and 3 were sacrificed on day 4, 5 and 8 after start of GCV treatment due to dramatic weight loss and revealed severe central lobular necrosis (arrow heads). Representative liver histology slices of GCV treated mice are shown. Total liver tissue was fixed in 4% formalin for six days. Fixed tissue was paraffin embedded and 3 µm slices were haematoxylin and eosin stained, Scale bars represent 200 µm.

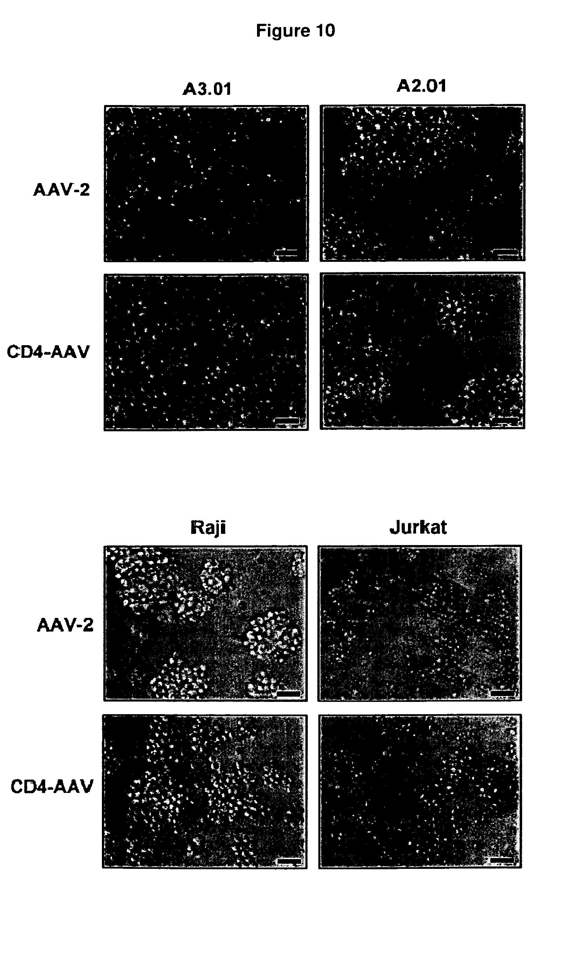

**Figure 10** shows that CD4-AAV selectively transduces CD4-positive T-cells. CD4-positive (A3.01) and CD4-negative (A2.01, Raji, Jurkat) lymphocyte cell lines were transduced with CD4-AAV or AAV-2 (GOI=300,000, respectively). 48 hours later, cells were analyzed by fluorescence microscopy. Scale bars represent 200 µm,

### Detailed description of the invention

The inventors developed recombinant AAV vector particles displaying high-affinity ligands, which can be used for cell-type specific gene delivery during therapeutic applications and applications in basic research since they provide high-cell type selectivity or rather high targeting specificity allowing restricted biodistribution and safe gene transfer and/or delivery of genes or rather transgenes.

The term "gene transfer" or "delivery of genes" according to the present invention is attempted by the use of viral methods, since viruses have developed efficient strategies to deliver inherent DNA or RNA to the host cells ("transduction"), so as to transform the host and promote expression (e.g. transcription and translation) of the introduced sequence. To date, several human or animal viruses are modified in order to be used as such vehicles for transduction, i.e. as "vectors" or "vector particles", including Adenovirus, Adeno-Associated Virus (AAV) and Retrovirus, wherein the present invention is limited to the use of AAV. Thus, according to the present invention, the term "AAV" refers to the Adeno-Associated Virus itself or to derivatives thereof including recombinant AAV vector particles, wherein the term "AAV wild-type particle" designates the Adeno-Associated Virus as it occurs in nature. Furthermore, as used herein, the term "AAV" or rather "recombinant AAV vector particle" and "AAV wild-type particle" includes the twelve different serotypes, which have been isolated from human (AAV of serotypes 2, 3, 5, 6, and 9) and non-human primate samples (AAV of serotypes 1, 4, 7, 8, 10), and AAV of serotypes 11 and 12. Thus, the term e.g. "AAV of human serotype 2" or "AAV of serotype 2" (rather than "AAV-2" or "AAV-2 particle") refers to a wild-type Adeno-Associated Virus of serotype 2 that does not comprise any recombination. The present invention is not limited to an AAV vector particle of any specific serotype, however, can be exploited for all human and non-human AAV serotypes, preferably for the human serotypes, more preferably for AAV of human serotypes 2, 5 and 8, most preferably for AAV of human serotype 2, wherein the "recombinant AAV vector particle" and the "AAV wild-type particle" correspond with each other in their serotype if they co-occur.

In one embodiment of the present invention, the present invention refers to a recombinant AAV vector particle (or rather "DARPin-proficient particle"), wherein the differences to the AAV wild-type particle are based on features (a) to (d) as mentioned above, wherein in another embodiment of the present invention, the differences to the AAV wild-type particle are based on feature (a) only. In the latter case, the recombinant AAV vector particle comprises an AAV-wild-type capsid and, thus, is designated as "recombinant AAV vector particle negative control". Since such negative control preferably comprises the AAV-wild-type capsid of AAV of serotype 2, it is also designated as "AAV-2" or as "AAV-2 particle" (rather than "AAV of human serotype 2" or "AAV of serotype 2").

Thus, the differences of the "recombinant AAV vector particle" to the AAV wild-type particle are initially based on a recombination, wherein the term "recombination" as used in the present invention means that the recombinant AAV vector particle and the recombinant AAV vector particle negative control comprises at least one heterologous polynucleotide, i.e. at least one polynucleotide other than the wild-type AAV genome, such as a packaged gene or rather transgene to be delivered to a cell, preferably to a mammalian cell (see feature (a)). In this context, the term "at least one packaged transgene" comprises a packaged transgene that encodes for a therapeutic gene and/or a packaged transgene that encodes for a marker/reporter gene, i.e. a packaged transgene that encodes for a therapeutically active product and/or a packaged transgene that encodes for a marker/reporter protein.

The "transgene" according to the present invention is a gene or genetic material that has been transferred from one organism to another. In more detail, the term "transgene" as used herein describes a segment of a DNA containing a gene sequence that has been isolated from one organism and is introduced into a different organism by the gene transfer methods as described above. This non-native segment of DNA of the present invention may retain the ability to produce RNA or protein in the transgenic organism. In practical terms, according to the present invention, a transgene can be either a cDNA (complementary DNA) segment, which is a copy of mRNA (messenger RNA), or the gene itself. Preferably, the cDNA of the present invention has been processed to remove introns and more preferably, does not include the regulatory signals that are embedded around and in the gene, wherein the term "regulatory signals" refers to transcriptional regulatory sequences, such as promoters and enhancers.

In a preferred embodiment, the transgene is a gene that is to be transduced into and expressed by the targeted cell. Preferably, the transgene is a therapeutic gene, i.e. a transgene encoding for a therapeutically active product, more preferably for a desired antibody gene or fragment thereof; a gene encoding an apoptosis-inducing protein, such as a suicide gene; a gene encoding an anti-apoptosis protein; a gene encoding a cytotoxic protein; a gene encoding a cytostatic protein; a tumor suppressor gene; an antibiotic resistance gene; a gene encoding siRNA; a HIV-inhibiting gene such as a gene encoding C peptides, which are efficient inhibitors of HIV-1 entry, or encoding shRNA for proteases or for HIV co-receptors; a gene encoding an angiogenic factor; a gene encoding a neuro-protective factor; a gene encoding a viral or bacterial antigen; a gene encoding an anti-viral protein; a gene encoding a tumoral antigen or rather tumor associated antigen; a gene encoding a cytokine, such as interferon, interleukin, colony-stimulating factor, tumor necrosis factor and chemokine; or a gene encoding a functional copy of a defective or mutated gene in a patient suffering from an inherited disease, such as from an inherited disease that is controlled by a single pair of genes ("monogenetic" or "monogenic disease"). The term "monogenetic disease" or "monogenic disease" includes but is not limited to diseases such as SCID (severe combined immunodeficiency), congenital disease, cystic fibrosis, Gaucher's disease, Huntington's disease, dysostosis, Hurler's disease, neurofibromatosis, sickle-cell anemia, Tay-Sachs disease, thalassemia, familial hypercholesterolemia, and fragile X syndrome.

In particular, the "suicide gene" will cause a cell to kill itself through apoptosis, wherein the suicide gene is selected from the group consisting of herpes simplex virus thymidine kinase (HSV-TK), which converts ganciclovir (GCV) into cytotoxic compounds, *Escherichia coli* cytosine deaminase, which allows the formation of a cytotoxic chemotherapeutic agent from a non-toxic precursor, Varicella-zoster virus thymidine kinase or deoxycytidine kinase. Thus, in another embodiment of the present invention, cell-type specific gene delivery by using the recombinant AAV particles of the present invention requires the additional administration of compounds that are converted by suicide gene products into cytotoxic compounds, such as treatment with GCV that is converted by HSV-TK. Furthermore, the "tumor suppressor gene" protects a cell from one step on the path to cancer, wherein the tumor suppressor gene is selected from the group consisting of p16, p27, p53, Rb1 (retinoblastoma 1 gene), PTEN, VHL, APC (adenomatous polyposis coli gene), CD95 (cluster of differentiation 95 gene), ST5 (suppression of tumorigenicity 5 gene), YPEL3, ST7 (suppressor of tumorigenicity 7 gene), and ST14 (suppressor of tumorigenicity 14 gene).

On the other hand, the term "transgene" of the present invention may additionally refer to a "marker/reporter gene" that is packaged in addition to or instead of the therapeutic gene ("and/or") in order to monitor *in vivo* and *in vitro* transduction and/or expression of the transgene. However, according to the present invention, a "marker/reporter gene" may be optionally used in applications of basic research only but not in therapeutic applications.

In particular, the term "marker/reporter gene" refers to the coding sequence or rather to the gene encoding for a fluorescent protein or an oxidative enzyme, in order to visualize the infection, i.e. to monitor successful transduction of the target cell or target tissue based on the expression of the marker/reporter gene. Preferred fluorescent proteins are the green fluorescent protein (GFP) or derivatives thereof, wherein such derivatives are selected from the group consisting of the 25-kDa enhanced GFP (EGFP), blue fluorescent protein derivatives (e.g. EBFP, EBFP2, Azurite, or mKala-ma1), cyan fluorescent protein derivatives (e.g. ECFP, Cerulean, or CyPet) and yellow fluorescent protein derivatives (e.g. YFP, Citrine, Venus, or YPet), wherein EGFP is the most preferred derivative. A preferred oxidative enzyme is the firefly luciferase.

The transgene, i.e. the therapeutic and/or marker/reporter gene of the present invention is packaged into the recombinant AAV vector particle, more specifically, is inserted into a "vector plasmid". In the case that a therapeutic gene and a marker/reporter gene is packaged into the recombinant AAV vector particle, the therapeutic gene and the marker/reporter gene can be inserted into the same vector plasmid. In a preferred embodiment of the present invention, the respective coding sequences are under the control of a promoter, preferably under the control of the spleen focus forming virus (SFFV) promoter, wherein the promoter-gene-construct is framed by repetitive sequences, such as inverted terminal repeats (ITRs), which serve as packaging signals (Fig. 1 a).

Furthermore, the differences of the "recombinant AAV vector particle" to the AAV wild-type particle are based on the mutation of the AAV capsid protein VP2 in order to allow cell-type specific gene delivery, wherein the mutation is based on a cell-type specific DARPin that is fused to the N-terminus of the AAV capsid protein VP2 ("DARPin-VP2 fusion protein" or "DARPin-VP2 fusion construct"). In particular, the term "cell-type specific DARPin" refers to designed ankyrin repeat proteins, that are specific for at least one surface receptor, preferably one surface receptor, that is expressed on the target cell or target tissue (see feature (b)).

In general, ankyrin repeat proteins are built from a single structural motif, which is assembled into a protein domain. Due to the relatively high sequence homology within these ankyrin repeats, a consensus sequence module may be deduced from the natural ankyrin repeat proteins, identifying putative structure-determining framework residues and interaction-mediating binding residues. A synthetic consensus repeat module of the ankyrin repeat protein family may be assembled into complex libraries of designed ankyrin repeat proteins, from which binding proteins may be isolated that show high expression yields, stabilities and affinities in the nanomolar range (Zahnd, C. et al., J. Mol. Biol., 2007, 369, 1015-1028).

Thus, the DARPins according to the present invention are high affinity multi-domain protein ligands with a molecular mass of about 14 kDa. The binding interface is flanked by two capping repeats and comprises 50-200, preferably 100-120 amino acids. Furthermore, they are characterized by high expression yields, which range from 100-300 mg/l soluble protein, preferably 200 mg/l soluble protein and provide a high thermodynamic stability, preferably 5-25 kcal/mol, more preferably 9.5-21 kcal/mol. They do not contain cysteines so that disulfide bonds are absent. Moreover, they are characterized by low nanomolar affinities along with high cell-type selectivity in order to avoid reactions with non-target cells or tissues, wherein the association rates are in the typical range of protein-protein interactions (preferably 10⁵-10⁶ M⁻¹s⁻¹) and the dissociation rates are in the range of 10⁻² to 2·10⁻³ s⁻¹. Up to now, DARPins have already been used as targeting domain, i.e. for pseudotyping of viral vectors, however, only in the combination with lentiviral vector particles (EP 1 975 239 A1 and Münch, R. C., Mol. Ther., 2011, 19, 686-693). The combination of DARPins with AAV vector particles, such as disclosed in the present invention, has not been described in the prior art and provides the following advantages with regard to the combination with lentiviral vecor particles: Due to their substantially smaller size as compared to lentiviral vector particles, AAV vector particles show much higher diffusion rates in tissue and therefore possess the ability to transduce large areas of tissue upon local injection. Moreover, AAV vector particles do not integrate the transfer vector into the cellular genome and, therefore, are not prone to insertional oncogenesis as observed with retro- and lentiviral vectors in clinical trials. Furthermore, it is expected that AAV vector particles are able to cross the blood-brain barrier, which would result in a broader application than observed for lentiviral vector particles. Due to their lower immunogenicity as compared to lentiviral vector particles and the possibility to transfer the system of the present invention to other AAV serotypes, the application of AAV vector particles is broader than of lentiviral vector particles. Thus, the cell-type specific DARPin of the present invention is specific for at least one surface receptor, preferably for one surface receptor that is expressed on the target cell or target tissue.

Only the igem team Freiburg recently suggested a recombinant, modularized, BioBrick-compatible AAV "Virus Construction Kit" for therapeutic application (Virus Construction Kit - The Manual, Freiburg Bioware igEM 2010, p.1-218). In such kit, AAV viral particles have been created with the surface exposed DARPin E_01, wherein the AAV genome was converted to BioBrick format, the targeting peptides were fused to the N-terminus of VP2, the essential natural receptor binding site was knocked-out, and 100% of the VP2-start codon was replaced by mutation, wherein the igem team Freiburg inserted the mutation at the same position but used another codon for the inserted alanine. However, as compared to the invention of the present application, the igem team Freiburg did not show recombinant AAV vector particles, which display high-affinity ligands exemplified for cell-type specific DARPin that specifically transduce target cells or target tissues, which express the respective target receptor. Furthermore, the igem team Freiburg fused the targeting peptide via a linker to the VP2 protein and did not purify the AAV vector particles by using an iodixanol gradient and/or a His-tag, wherein these differences may be the reason that the igem team Freiburg observed an incomplete restriction of AAV vector tropism only and transduction rates that were only slightly above the detection limit. Based on the low transduction efficacy achieved by the igem team Freiburg, it is not surprising that they did not show that their AAV vector particles can be used in combination with a transgene encoding e.g. a therapeutically active product (in contrast to the results of the present invention).

Furthermore, owning to the lack of purification, the igem team Freiburg even formally missed to proof that indeed particles displaying DARPin ligands have been formed (in contrast to results of the present invention). In addition, the igem team Freiburg did not show that DARPins are accessible on the viral capsid and that DARPin as DARPin-VP2 fusion protein mediates selective cell transduction (in contrast to results of the present invention). The present invention is further distinguished from the invention of the igem team Freiburg, as the present invention clearly shows the capability of DARPin-tagged viral vectors to transduce receptor-positive cells *in vivo* following systemic application, efficacy and specificity *in vivo* and *ex vivo* and the ability to purify the tagged particles by affinity chromatography.

The cell-type specific DARPins of the present invention, which are specific for at least one surface receptor, preferably for one surface receptor that is expressed on the target cell or target tissue, are displayed by the AAV capsid protein, which means the AAV capsid protein VP2 is modified, wherein its N-terminal region or rather N-terminus serves as the platform to display DARPin.

In a preferable embodiment of the present invention, the cell-type specific DARPin is specific for a single surface receptor that is expressed on the target cells or target tissues. Preferably, the DARPin is specific for HER2/*neu,* a receptor tyrosine kinase that is over-expressed on human cancer cells, preferably DARPin 9.29, or for human CD4, a co-receptor that assists the T cell receptor with an antigen-presenting cell, preferably DARPin 55.2 (see below).

Moreover, the differences of the "recombinant AAV vector particle" of the present invention to the AAV wild-type particle are based on the mutation of the native VP2-start codon in order to avoid expression of native VP2 by deactivation the transcription of the VP2-gene (see feature (c)). Preferably, the mutation is a mutation that results in a functional deletion. More preferably, the mutation does not change the reading frame, preferably a point mutation, more preferably a point mutation in the unusual start codon ACG to ACC.

Furthermore, the differences of the "recombinant AAV vector particle" of the present invention to the AAV wild-type particle are based on a capsid, wherein the essential binding site for the natural receptor of the AAV capsid proteins VP1, VP2 and VP3 is mutated (see feature (d)). As used herein, the term "natural receptor" refers, for example, to heparan sulfate proteoglycan (HSPG), which has been shown to be the primary cellular receptor for AAV of serotype 2; to N-linked sialic acid containing glycans, which have been shown to be the primary cellular receptor for AAV of serotypes 1, 5 and 6; to O-linked sialic acid containing glycans, which has been shown to be the primary cellular receptor for AAV of serotype 4 and 9; to αVβ5 integrin, α5β1 integrin, CD9, and hepatocyte growth factor receptor, which act as secondary receptors or rather co-receptors for AAV of serotype 2; to basic fibroblast growth factor receptor and 37/67 kDa laminin receptor (LamR), which act as secondary receptors or rather co-receptors for AAV of serotypes 2, 3, 8, and 9; and/or to the platelet derived growth factor receptor (PDGFR), which act as secondary receptors or rather co-receptors for serotype AAV-5.

The term "essential binding site" refers to at least two amino acid residues in the natural receptor binding site, i.e. in the common VP3 region of each capsid protein, which is "common", since the regions encoding the VP1 and VP2 proteins represent N-terminal extensions of the region encoding the VP3 protein. Thus, the reading frames of the regions encoding VP1, VP2 and VP3 are overlapping, so that the mutation in the essential natural receptor binding site is present in all of the three capsid proteins within their common VP3 region. In the case of AAV of serotype 2, the primary receptor binding motif is constituted by five positively charged amino acid residues located in the common VP3 region of each capsid protein. Thus, in a preferred embodiment, VP1, VP2 and VP3 of AAV of serotype 2 are mutated at positions 585 and/or 588, respectively, more preferably by the substitutions R585A and/or R588A, since these residues represent the main or rather essential residues involved in HSPG-binding (Fig. 1). Following, a mutation at the sites mentioned above leads to a de-targeting of the AAV vector from its natural target cells or tissues, e.g. from liver after systemic application.

Corresponding positions to position 585 of AAV of serotype 2 is position 586 of AAV of serotypes 1, 3, 4 and 5, and the corresponding position to position 588 of AAV of serotype 2 is position 590 of AAV of serotypes 1, 3, 4 and 5. Thus, using other serotypes than serotype 2, the term "essential binding site" may refer to other positions than 585 and/or 588.

Such recombinant AAV vector particles of the present invention can be used for applications in basic research and therapeutic applications. In particular, the term "basic research" of the present invention refers to research that is carried out to increase understanding of fundamental principles and is not intended to yield immediate commercial benefits, wherein the term "therapeutic applications" of the present invention is based on the use of the recombinant AAV vector particles for transducing a specific target cell or target tissue with a gene or rather transgene product of a desired protein that, if expressed in the target cell or target tissue, leads to the prevention or the treatment of a particular medical condition.

Such "target cells" or "target tissues" of the present invention are characterized by the fact that they contain the surface receptor for the respectively used DARPin, i.e. can be described as "receptor-positive cells" or "receptor-positive tissues". Preferably, the DARPin is specific for HER2/*neu*, a receptor tyrosine kinase that is over-expressed on human cancer cells, preferably DARPin 9.29, or for human CD4, a co-receptor that assists the T cell receptor with an antigen-presenting cell, preferably DARPin 55.2. Thus in one preferred embodiment of the present invention, the HER2/*neu*-specific DARPin (preferably DARPin 9.29) allows the therapy and/or diagnosis of HER2/*neu*-positive tumors and/or the labeling of HER2/*neu*-positive tumor cells and in another preferred embodiment, the CD4-specific DARPin (preferably DARPin 55.2) allows the therapy and/or diagnosis of the human immunodeficiency virus (HIV).

In more detail, the term "HER2/*neu*" generally refers to an epidermal growth factor, more specifically to a type-I receptor tyrosine kinase, which is highly expressed on breast, ovarian, colon and pancreatic cancer cells but shows low level or no expression on all normal human tissue. In total, six DARPins exhibiting distinct affinities for HER2/*neu* are known: DARPin 9.29, 9.26, 9.16, 9.01, H14R, and G3 (Steiner, D. et al., J. Mol. Biol., 2008, 382, 1211-1227; Münch, R. C. et al., Mol. Ther., 2011, 19, 686-693). Thus, as used herein, the term "HER2/*neu*-specific DARPin" refers to DARPin 9.29, 9.26, 9.16, 9.01, H14R, or G3, preferably to DARPin 9.29, wherein in one preferred embodiment of the present invention, the recombinant AAV vector particles comprise a HER2/*neu*-specific DARPin as targeting domain ("Her2-AAV", AAV-Her2" or "Her2-AAV particles). Therefore, Her2-AAV particles are re-targeted to HER2/*neu*-positive tumor cells, wherein in a more preferred embodiment, the recombinant AAV vector particle is re-targeted to HER2/*neu*-positive breast, ovarian, colon or pancreatic cancer cells. For example, Her2-AAV particles are re-targeted to the Chinese hamster ovary cell lines CHO-Her2-k6, CHO-Her2-k7, CHO-Her2-K13 or to the fibrosarcoma cell line HT1080, which stably express HER2/*neu*, to the ovarian cancer cell line SK-OV-3 naturally over-expressing HER2/*neu* (Münch, R.C. et al., Mol. Ther., 2011, 19, 686-693), to the human breast carcinoma or rather adenocarcininoma cell lines BT-474, SK-BR3, MDA-MB-361 or MDA-MB-453.

Moreover, the term "CD4" generally refers to the cluster of differentiation 4, a glycoprotein that is expressed on the surface of T helper cells, monocytes, macrophages, and dendritic cells. With regard to HIV, HIV infection leads to a progressive reduction in the number of T cells expressing CD4. HIV-1 uses CD4 to gain entry into host T-cells and achieves this by binding of the viral envelope protein known as gp120 to CD4. The binding to CD4 creates a shift in the conformation of gp120 allowing HIV-1 to bind to a co-receptor expressed on the host cell. These co-receptors are chemokine receptors CCR5 or CXCR4, wherein which of these co-receptor is used during infection is dependent on whether the virus is infecting a macrophage or T-helper cell. Following a structural change in another viral protein (gp41), HIV inserts a fusion peptide into the host cell that allows the outer membrane of the virus to fuse with the cell membrane (Kwong, P. D. et al., Nature, 393, 648-659). In total, six different DARPins exhibiting distinct affinities for CD4 are known: DARPin 3.1, 23.2, 27.2, 29.2, 55.2, and 57.2 (Schweizer, A. et al., PLoS Pathog., 2008, 4, e1000109).

Thus, as used herein, the term "CD4-specific DARPin" refers to DARPin 3.1, 23.2, 27.2, 29.2, 55.2, or 57.2, preferably to DARPin 55.2, wherein in one preferred embodiment of the present invention, the recombinant AAV vector particles comprise a CD4-specific DARPin as targeting domain ("CD4-AAV", "AAV-CD4", or "CD4-AAV particles"). Therefore, according to the present invention, CD4-AAV particles are re-targeted to CD4-positive cells, for example to T-lymphocytes expressing CD4, i.e. to CD4-positive lymphocytes, preferably to JM, CCRF, CEM, U937, HL60, and A3.01 T cells, more preferably A3.01 T cells and, thus, can be used for therapy and/or diagnosis of HIV. However, such recombinant AAV vector particle can be also used for therapy and/or diagnosis of neoplasms that derive from T helper cells; for therapy and/or diagnosis of autoimmune diseases, such as vitiligo and type I diabetes mellitus; and for CD4 immunohistochemistry on tissue biopsy samples to identify peripheral T cell lymphoma and related malignant conditions.

However, the present invention is not limited on the use of the HER2/*neu-*specific or CD4-specific DARPins but refers to recombinant AAV vector particles which display high-affinity ligands exemplified by cell-type specific DARPin that specifically transduce any type of target cell or target tissue, which express the respective target receptor. Thus, the term "therapeutic application" of the present invention does not only include HER2/*neu*- or CD4-associated diseases but also includes the preparation of a medicament comprising the recombinant AAV vector particles of the present invention for the treatment or prevention of at least one condition in a subject, wherein the condition is selected from the group comprising a chronic infection; an inherited monogenetic disease, including SCID, congenital disease, cystic fibrosis, Gaucher's disease, Huntington's disease, dysostosis, Hurler's disease, neurofibromatosis, sickle-cell anemia, Tay-Sachs disease, thalassemia, familial hypercholesterolemia, and fragile X syndrome; a cardiovascular disease; a neurodegenerative disease; cancer; HIV; Alzheimer disease; Parkinson disease; diabetes; a neuroinflammatory disease; a rheumatic disease; an autoimmune disease; adipositas; acute lymphoblastic leukemia; myeloid leukemia; renal carcinoma; and disorders related to endothelialization or re-endothelialization. In yet another embodiment, the cancer is selected from the group comprising leukemia, myeloblastic, promyelocytic, myelomonocytic, monocytic, erythroleukemia, chronic myelocytic (granulocytic) leukemia, and chronic lymphocytic leukemia, lymphoma, e.g. Hodgkin's disease and non-Hodgkin's disease, fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilms' tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia, prostate cancer, prostatitis, benign prostatic hypertrophy, benign prostatic hyperplasia (BPH), prostatic paraganglioma, prostate adenocarcinoma, prostatic intraepithelial neoplasia, prostato-rectal fistulas, and atypical prostatic stromal lesions.

Furthermore, the term "therapeutic application" includes gene therapy for the treatment or prevention of at least one of the conditions of above, wherein the term "gene therapy" can be broadly defined as the concept of directed introduction of foreign genetic material into a cell, tissue or organ for correction of defective genes with the goal to improve the clinical status of a patient. As used herein, the term "gene therapy" only refers to "somatic therapy" and not to "germ line therapy", which would induce heritable changes passed from generation to generation, wherein the somatic therapy restricts the therapeutic effect to the treated individual. The somatic therapy of the present invention can be further discriminated by a fast and easy to perform direct gene transfer to the organism ("*in vivo*") or a sophisticated but more specific and controllable gene transfer to explanted cells or tissues ("*ex vivo*" or "*in vitro*")*,* which are re-implanted after treatment. Thus, the recombinant AAV vector particle of the present invention is administered *in vivo,* i.e. systemically, or *ex vivo*

However, as the AAV capsid protein VP2 is not essential for capsid assembly, the "recombinant AAV vector particle preparations" of the present invention may consist of DARPin-proficient and, in exceptional, undesirable cases, of DARPin-deficient particles. The "DARPin-proficient particles" represent the "recombinant AAV vector particles" of the present invention as described above, wherein the latter, i.e. the "DARPin-deficient particles" are distinguished from these "recombinant AAV vector particles" or rather "DARPin-proficient particles" in that their differences to the AAV wild-type particle are based on features (a), (c) and (d) only. Thus, the DARPin-deficient particles are composed of the AAV capsid proteins VP1 and VP3, which are ablated in binding to the natural receptor, e.g. HSPG, however, are deficient in the DARPin-VP2 fusion protein (feature (b)), i.e. the targeting ligand. Although non-infectious *in vitro,* DARPin-deficient particles may transduce off-target tissue, such as heart and lung tissue, although they are deficient in binding to their natural receptor and do not comprise the DARPin-VP2 fusion protein ("off-targeting", Example 5, Fig. 1 c, Fig. 6). Thus, in a preferred embodiment of the present invention, a removable His-tag is fused to the N-terminal end of the DARPin-VP2 fusion protein of feature (b) and incorporated into the recombinant AAV vector particles of the present invention ("recombinant AAV vector particles^{His}"), such as into Her2-AAV particles ("Her2-AAV^{His}"), in order to reduce off-targeting effects, wherein the recombinant AAV vector particles^{His}, such as Her2-AAV^{His}, can be eluted in distinct fractions using His-tag purification methods, such as the column purification as described in Example 1, wherein the His-tag can be removed after purification by enzymatic cleavage. These purified recombinant AAV vector particles^{His}, such as purified Her2-AAV^{His} vector particles, are able to transduce exclusively target cells or rather target tissue, such as tumor tissue rather than heart and lung tissue e.g. upon intravenous injection into tumor bearing subjects, such as mammals.

In summary, the present invention refers to a recombinant AAV vector particle or rather DARPin-proficient particle, wherein the differences to the AAV wild-type particle are based on features (a) to (d). In contrast, the recombinant AAV vector particle itself contains features (a), (b) and (d) only, which means that the mutated VP2-start codon (feature (c)) is not present in the recombinant AAV vector particle but represents a necessary feature for generation the differences of the recombinant AAV vector particle to the AAV wild-type particle. Thus, the present invention claims a "recombinant AAV vector particle, wherein the differences to the AAV wild-type particle are based on the following features: (a), (b), (c), and (d)".

More specifically, the recombinant AAV vector particles of the present invention are generated using the adenovirus-helper free AAV packaging strategy (Xiao, X. et al., J. Virol., 1998, 72, 2224-2232), wherein the methods for the construction and generation of the recombinant AAV vector particles of the present invention are mainly based on a transfection method, which requires the use of:
(i) a vector plasmid comprising the at least one packaged transgene, as described in feature (a),
(ii) a plasmid encoding the DARPin-VP2 fusion protein ("pDARPin-VP2"), wherein VP2 comprises the mutations in the VP2-start codon and in the essential binding site as described in features (c) and (d), and wherein a removable His-tag is optionally fused to the N-terminal end of the DARPin-VP2 fusion protein,
(iii) an AAV helper plasmid, preferably "pRCVP2koA", encoding for AAV non-structural proteins, preferably viral Rep proteins (preferably the non-structural Rep proteins Rep78, Rep68, Rep52, Rep40) and AAP (assembly activating protein), and for the capsid proteins VP1, VP2 and VP3, wherein the AAV helper plasmid comprises the mutation in the VP2 start codon and in the essential natural receptor binding site as described in features (c) and (d), and
(iv) at least one Ad helper plasmid, preferably "pXX6", encoding for genes possessing Ad helper functions, preferably for E2A, E4 and VA RNA genes.

In more detail, by using the vector plasmid of step (i), the therapeutically active product and/or the marker/reporter protein is provided as vector genome, by using the plasmid pDARPin-VP2 of step (ii), the DARPin-VP2 fusion protein, comprising the mutated capsid protein VP2 and the cell-type specific DARPin, is expressed, wherein the expression of the capsid protein VP2 wild-type ("unmodified VP2") is inhibited; by using the AAV helper plasmid of step (iii), the viral Rep proteins, preferably the non-structural Rep proteins Rep78, Rep68, Rep52, Rep40, AAP, and the capsid proteins VP1 and VP3 are expressed, wherein VP1 and VP3 are not able to bind to the natural receptor; by using the at least one Ad helper plasmid of step (iv), proteins possessing Ad helper functions are expressed.

Thus, in a preferred embodiment of the present invention, the recombinant AAV vector particles are produced by co-transfection of a human cell line, preferably HEK-293, with the vector plasmid as mentioned in (i), the plasmid pDARPin-VP2 as mentioned in (ii), the AAV helper plasmid, preferably pRCVP2koA, as mentioned in (iii), and the at least one Ad helper plasmid, preferably pXX6, as mentioned in (iv), wherein these plasmids are preferably mixed in an equimolar ratio with a cationic polymer, preferably polyethylene-imine (PEI) or poly-I-lysine, more preferably PEI, for use as attachment promoter, transfection reagent or for CO₂ capture, as previously described for lentiviral vectors (Anliker, B., et al., Nat. Meth., 2010, 7, 929-935). 48 hours after transfection, cells are harvested, pelleted by centrifugation and lysed. Cell lysate is treated by Benzonase (Sigma), pre-cleared by low speed centrifugation and subjected to iodixonal density gradient centrifugation as described previously (Boucas, J. et al., J. Gene Med., 2009, 11, 1103-1113). Vectors or rather vector particles can contain a self-complementary or single stranded vector genome conformation.

Moreover, the recombinant AAV vector particle negative control, preferably using AAV of serotype 2 and then designated as "AAV-2" or "AAV-2 particles", is also generated using the adenovirus-helper free AAV packaging strategy (Xiao, X. et al., J. Virol., 1998, 72, 2224-2232). Thus, preferably HEK-293 cells are co-transfected with the plasmids pXX6, pRC (Girod, A. et al., Nat. Meth., 1999, 1052-1056) and the at least one vector plasmid as mentioned in (i), wherein these plasmids are preferably mixed in an equimolar ratio with a cationic polymer, preferably polyethylene-imine (PEI) or poly-I-lysine, more preferably PEI. 48 hours after transfection, cells are harvested, pelleted by centrifugation and lysed. Cell lysate is treated by Benzonase (Sigma), pre-cleared by low speed centrifugation and subjected to iodixonal density gradient centrifugation. All vectors or rather vector particles can contain a self-complementary or single-stranded vector genome conformation.

In a more preferred embodiment of the present invention, the construction and generation of the plasmid "pDARPin-VP2" is based on the targeting construct pGFP-VP2 (Lux, K. et al., J. Virol., 2005, 79, 11776-11787) encoding the VP2 protein fused to EGFP at its N-terminus, wherein the VP2 start codon is mutated, preferably by point mutation, as described in feature (c). Thus, the construction and generation of the plasmid "pDARPin-VP2" is based on:
(1) substituting the EGFP-coding sequence for that of the cell-type specific DARPin of the present invention in pGFP-VP2 in order to produce the DARPin-VP2 fusion protein, wherein a removable His-tag can be optionally fused to the N-terminal end of the DARPin-VP2 fusion protein.
(2) ablating the binding of the capsid protein VP2 to its natural receptor in the DARPin-VP2 fusion protein, as described in feature (d).

### Example 1: Methods

**DARPin coding sequences.** The nucleotide sequences of the CD4-specific DARPins 3.1, 23.2, 27.2, 29.2, 55.2 and 57.2 originate from Schweizer, A. et al., PLoS Pathog., 2008, 4, e1000109 and the EMBL Nucleotide Sequence Data Base (www.ebi.ac.uk/embl) and, thus, are available under the accession numbers AM997261, AM997265, AM997267, AM997268, AM997269, and AM997270, respectively (direction 5' → 3').
DARPin 3.1 - AM997261 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGATGTT
AACGCCTTTGACGCTAATGGTATTACTTCGCTGCACCTGGCTGCTATGGAGGGTCACCTG
GAAATCGTTGAAGTTCTGCTGAAGTACGGTGCTGACGTTAACGCTTGGGACTCTTGGGGT
TATACTCCGCTGCACCTGGCTGCTGCTTATGGTCACCTGGAAATCGTTGAAGTTCTGCTG
AAGAAAGGTGCTGACGTTAACGCTTCTGACATTGATGGTTGGACTCCGCTGCACCTGGCT
GCTTCTAATGGTCACCTGGAAATCGTTGAAGTTCTGCTGAAGCACGGCGCTGACGTTAAC
GCTCAGGACAAATTCGGTAAGACCGCTTTCGACATCTCCATCGACAACGGTAACGAGGAC
CTGGCTGAAATCCTGCAAAAGCTTAATTAA
DARPin 23.2 - AM997265 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTACTGACACTCTTGGTCGTACTCCGCTGCACATGGCTGCTGCTTGGGGTCACCTG
GAAATCGTTGATGTTCTGCTGAAGCACGGTGCTGACGTTAACGCTATTGAAGAGGTTGGT
ATGACTCCGCTGCACCTGGCTGCTTTTCTTGGTCACCTGGAAATCGTTGAAGTTCTGCTG
AAGAGCGGTGCTGACGTTAACGCTCAGGACAAATTCGGTAAGACCGCTTTCGACATCTCC
ATCGACTACGGTAACGAGGACCTGGCTGAAATCCTGCAAAAGCTTAATTAA
DARPin 27.2 - AM997267 
ATGAGAGGATCGCATCACCATCACCATCACGGATCCGACCTGGGTAAGAGACTGCTGGAA
GCTGCTCGTGCTGGTCAGGATGACGAAGTTCGTATCCTGATGGCTAACGGTGCTGACGTT
AACGCTAAGGACGATGAGGGTCGTACTTCGCTGCACCTGGCTGCTCGTGAGGGTCACCTG
GAAATCGTTGAAGTTCTGCTGAAGCACGGTGCTGACGTTAACGCTCAGGACTGGTATGGT
TCTACTCCGCTGCACCTGGCTGCTGCTTGGGGTCACCTGGAAATCGTTGAAGTTCTGCTG
AAGAACGTTGCTGACGTTAACGCTATGGACGGTGATGGTTCTACTCCGCTGCACCTGGCT
GCTCATTATGCTCACCTGGAAGTCGTTGAAGTTCTGCTGAAGAACGGTGCTGACGTTAAC
GCTCAGGACAAATTCGGTAAGACCGCTTTCGACATCTCCATCGACAACGGTAACGAGGAC
CTGGCTGAAATCCTGCAAAAGCTTAATTAA
DARPin 29.2 - AM997268 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTAAGGACGATGAGGGTCGTACTCCGCTGCACCTGGCTGCTCGTGAGGGTCACCTG
GAAATCGTTGAAGTTCTGCTGAAGCACGGTGCTGACGTTAACGCTCAGGACTGGTATGGT
 
TCTACTCCGCTGCACCTGGCTGCTGCTTGGGGTCACCTGGAAATCGTCGAAGTTCTGCTG
AAGAACGTTGCTGACGTTAACGCTATGGACGATGATGGTTCTACTCCACTGCACCTGGCT
GCTCATTATGCTCACCTGGAAGTCGTTGAAGTTCTGCTGAAGAGCGGTGCTGACGTTAAC
GCTCANGACAAATTCGGTAAGACCGCTTTCGACATCTCCATCGACAACGGTAACGAGGAC
CTGGCTGAAATCCTGCAAAAGCTTAATTAA
DARPin 55.2 - AM997269 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTATGGACATTCTTGGTCTTACTCCGCTGCACCTGGCTGCTGTTGCTGGTCACCTG
GAAATCGTTGAAGTTCTGCTGAAGAACAGTGCTGACGTCAACGCTATTGACGAGGATGGT
GAGACTCCACTGCACCTGTCTGCTGCTATGGGTCACCTGAAAATCGTTGAAGTTCTGCTG
AAGCACGGTGCCGACGTTAACGCTCAGGACAAATTCGGTAAGACCGCTTTCGACATCTCC
ATCGACTACGGTAACGAGGACCTGGCTGAAATCCTGCAAAAGCTTAATTAA
DARPin 57.2 - AM997270

ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTATGGACCATTTTGGTTTACTCCGCTGCACCTGGCTGCTAAGGTTGGTCACCTG
GAAATCGTTGAAGTTCTGCTGAAGTACGGTGCTGACGTTAACGCTGATGACATGGATGGT
GAGACTCCGCTGCACCTGGCTGCTGCTATTGGTCACCTGGAAATCGTTGAAGTTCTGCTG
AAGAACGGTGCTGACGTTAACGCTCATGACACTTGGGGTTTACTCCGCTGCACCTGGCT
GCTTCTTATGGTCACCTGGAAATCGTTGAAGTTCTGCGGAAGTACGGTGCTGACGTTAAC
GCTCNNGACAAATTCGGTGAGACCACTTTCGACATCTCCATCGACAACGGTAACGAGGAC
CTGGNTGAAATCCTGCAAAAGCTTAATTAA

The nucleotide sequences of the HER2/*neu*-specific DARPin 9.29, 9.26, 9.16, 9.01, H14R, and G3 originate from Steiner, D. et al., J. Mol. Biol., 2008, 382, 1211-1227 and Münch, R. C. et al., Mol. Ther., 2011, 19, 686-693 (direction 5' → 3').
DARPin 9.01 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTGAGGACGAGGGTCGTACTCCGCTGCACCTGGCTGGTCACCTGGAAATCGTTGAA
GTTCTGCTGAAGAACGGTGCTGACGTTAACGCTGCTGACGCTTCTGGTCATACTCCGCTG
CACCTGGCTGCTCATCTTGGTCACCTGGAAATCGTTGAAGTTCTGCTGAAGCACGGTGCT
GACGTTAACGCTTGGGACAAGTTTGGTGTTACTCCGCTGCACCTGGCTGCTGATCATGGT
CACCTGGAAATCGTTGAAGTTCTGCTGAAGAACGGTGCTGACGTTAACGCTCAGGACAAA
TTCGGTAAGACCGCTTTCGACATCTCCATCGACAACGGTAACGAGGACCTGGCTGAAATC
CTGCAAAAGCTTAATTAA
DARPin 9.16 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTCATGACTTTCATGGTCTTACTCCGCTGCACCTGGCTGCTGGTATGGGTCACCTG
GAAATCGTTGAAGTTCTGCTGAAGAACGGTGCTGACGTTAACGCTGTTGACACTGATGGT
ATTACTCTGCTGCACCTGGCTGCTTATTATGGTCACCTGGAAATCGTTGAAGTTCTGCTG
 
AAGCACGGTGCTGACGTTAACGCTCATGACTATGCTGGTTCTACTCCGCTGCACCTGGCT
GCTAATACTGGTCACCTGGAAATCGTTGAAGTTCTGCTGAAGAACGGTGCTGACGTTAAC
GCTCAGGACAAATTCGGTAAGACCGCTTTCGACATCTCCATCGACAACGGTAACGAGGAC
CTGGCTGAAATCCTGCAAAAGCTTAATTAA
DARPin 9.26 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTAAGGACTTTATGGTATTACTCCGCTGCACCTGGCTGCTGCTTATGGTCACCTG
GAAATCGTTGAAGTTCTGCTGAAGCACGGTGCTGACGTTAACGCTCATGACTGGAATGGT
TGGACTCCGCTGCACCTGGCTGCTAAGTATGGTCACCTGGAAATCGTTGAAGTTCTGCTG
AAGCACGGTGCTGACGTTAACGCTATTGACAATGCTGGTAAGACTCCGCTGCACCTGGCT
GCTGCTCATGGTCACCTGGAAATCGTTGAAGTTCTGCTGAAGTACGGTGCTGACGTTAAC
GCTCAGGACAAATTCGGTAAGACCGCTTTCGACATCTCCATCGACAACGGTAACGAGGAC
CTGGCTGAAATCCTGCAAAAGCTTAATTAA
DARPin 9.29 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTCATGACTTTTATGGTATTACTCCGCTGCACCTGGCTGCTAATTTTGGTCACCTG
GAAATCGTTGAAGTTCTGCTGAAGCACGGTGCTGACGTTAACGCTTTTGACTATGATAAT
ACTCCGCTGCACCTGGCTGCTGATGCTGGTCACCTGGAAATCGTTGAAGTTCTGCTGAAG
TACGGTGCTGACGTTAACGCTTCTGACCGTGATGGTCATACTCCGCTGCACCTGGCTGCT
CGTGAGGGTCACCTGGAAATCGTTGAAGTTCTGCTGAAGAACGGTGCTGACGTTAACGCT
CAGGACAAATTCGGTAAGACCGCTTTCGACATCTCCATCGACAACGGTAACGAGGACCTG
GCTGAAATCCTGCAAAAGCTTAATTAA
DARPin H14R 
ATGAGAGGATCGCATCACCATCACCATCACGGATCCGACCTGGGTAAGAAACTGCTGGAA
GCTGCTCGTGCTGGTCAGGACGACGAAGTTCGTATCCTGATGGCTAACGGTGCTGACGTT
AACGCTACTGACATTCATGGTCATACTCCGCTGCACCTGGCTGCTGCTATGGGTCACCTG
GAAATCGTTGAAGTTCTGCTGAAGAACGGTGCTGACGTTAACGCTAATGACTGGCGTGGT
TTTACTCCGCTGCACCTGGCTGCTCTTAATGGTCACCTGGAAATCGTTGAAGTTCTGCTG
AAGAACGGTGCTGACGTTAACGCTACTGACACTGCTGGTAATACTCCTCTGCACTTGGCT
GCTTGGTTTGGTCACCTGGAAATCGTTGAAGTTCTGCTGAAGAACGGTGCTGACGTTAAC
GCTCAGGACAAATTCGGTAAGACCGCTTTCGACATCTCCATCGACAACGGTAACGAGGAC
CTGGCTGAAATCCTGCAAAAGCTTAATTAA
DARPin G3

ATGAGAGGATCGCATCACCATCACCATCACGGATCCGACCTGGGTAAGAAACTACTGGAA
GCTGCCCGTGCTGGTCAGGACGACGAAGTTCGTATCCTGATGGCTAACGGTGCTGACGTC
AACGCTAAGGACGAGTATGGTCTTACTCCACTGTACCTGGCTACTGCTCATGGTCACCTG
GAGATCGTTGAAGTTCTGCTGAAGAACGGTGCTGACGTTAACGCTGTTGACGCTATTGGT
TTTACTCCGCTGCACCTGGCTGCTTTTATTGGTCACCTGGAAATCGCTGAAGTTCTGCTG
AAGCACGGTGCTGACGTTAACGCTCAGGACAAATTCGGTAAGACCGCTTTCGACATCTCC
ATCGGCAACGGTAACGAGGACCTGGCTGAAATCCTGCAAAAGCTTAATTAA

**Plasmids.** DARPin coding sequences were amplified by PCR using the primer pairs A1-for and A1-rev or A2-for and A2-rev, and were inserted into the plasmid pGFP-VP2 by sticky end ligation using Agel and BsrGl restriction sites:
A1-for: 5'-GGAAGGACCGGTATGGACCTGGGTAAGAAACTG-3',
A1-rev: 5'-CCCGGCCCTGTACAGATTAAGCTTTTGCAGGATTTC-3',
A2-for: 
5'-GGAAGGACCGGTATGGAACAAAAACTTATTTCTGAAGAAGATCTGA-
TCGAGGGAAGGGACCTGGGTAAGAAACTGCTG-3'
A2-rev: 5'-CCCGGCCCTGTACAGATTAAGCTTTTGCAGGATTTC-3'

In order to ablate natural HSPG-binding of AAV-2 capsids, R585 and R588 coding residues were mutated to alanine by site-directed mutagenesis (Boucas, J. et al., J. Gene Med., 2009, 11, 1103-1113). Three different AAV vector plasmids were generated (scLUC, scGFP and scHSV-TK). Briefly, the coding sequences for firefly luciferase GL4 (primer pair L1-for and L1-rev), for the spleen focus forming virus (SFFV) promoter (primer pair S1-for and S1-rev) were amplified by PCR:
L1-for: 
5’-TTATTGACTAGTTATTAATAGTAATCAATTACGGGGTCATTAGTTCA-
TAGCCCATATATAATGAAAGACCCCACCTGTAGG-3’,
L1-rev: 5'-GCCTCGAGCGGCCGCTTTACACGGCGATCTTGC-3',
S1-for: 
5’-TTATTGACTAGTTATTAATAGTAATCAATTACGGGGTCATTAGTTCA-
TAGCCCATATATAATGAAAGACCCCACCTGTAGG-3’,
S1-rev: 5'-AATGTCCGCGGTACCCAGCCCCGGGCGACTCAGTCAATC-3'.

Fragments were inserted into scAAV/EGFP (Hacker, U. T. et al., J. Gene Med., 2005, 7, 1429-1438) by sticky end ligation at SpeI/NotI or SpeI/KpnI restriction sites, respectively, resulting in scLUC and scGFP. The HSV-TK coding sequence (Funke, S. et al., Mol. Ther., 2008, 16, 1427-1436; was inserted into scLUC using the primer pair H1-for and H1-rev and restriction sites SacII/NotI:
HSV-TK coding sequence (direction 5' → 3') 
ATGGCTTCGTACCCCTGCCATCAACACGCGTCTGCGTTCGACCAGGCTGCGCGTTCTCGC
GGCCATAGCAACCGACGTACGGCGTTGCGCCCTCGCCGGCAGCAAGAAGCCACGGAAGTC
CGCCCGGAGCAGAAAATGCCCACGCTACTGCGGGTTTATATAGACGGTCCCCACGGGATG
 
GGGAAAACCACCACCACGCAACTGCTGGTGGCCCTGGGTTCGCGCGACGATATCGTCTAC
GTACCCGAGCCGATGACTTACTGGCGGGTGCTGGGGCTTCCGAGACAATCGCGAACATC
TACACCACACAACACCGCCTCGACCAAGGAGAAATATCGGCCGGGACGCGGCGGTGGTA
ATGACAAGCGCCCAGATAACAATGGGCATGCCTTATGCCGTGACCGACGCCGTTCTGGCT
CCTCATATCGGGGGGGAGGCTGGGAGCTCACATGCCCCGCCCCCGGCCCTCACCATCTTC
CTCGACCGCCATCCCATCGCCTTCATGCTGTGCTACCCGGCCGCGCGGTACCTTATGGGC
AGCATGACCCCCCAGGCCGTGCTGGCGTTCGTGGCCCTCATCCCGCCGACCTTGCCCGGC
ACCAACATCGTGCTTGGGGCCCTTCCGGAGGACAGACACATCGACCGCCTGGCCAAACGC
CAGCGCCCCGGCGAGCGGCTGGACCTGGCTATGCTGGCTGCGATTCGCCGCGTTTACGGG
CTACTTGCCAATACGGTGCGGTATCTGCAGTGCGGCGGGTCGTGGCGGGAGGACTGGGGA
CAGCTTTCGGGGACGGCCGTGCCGCCCCAGGGTGCCGAGCCCCAGAGCAACGCGGGCCCA
CGACCCCATATCGGGGACACGTTATTTACCCTGTTTCGGCCCCCGAGTTGCTGGCCCCC
AACGGCGACCTGTATAACGTGTTTGCCTGGGCCTTGGACGTCTTGGCCAAACGCCTCCGT
TCCATGCACGTCTTTATCCTGGATTACGACCAATCGCCCGCCGGCTGCCGGGACGCCCTG
CTGCAACTTACCTCCGGGATGGTCCAGACCCACGTCACCACCCCCGGCTCCATACCGACG
ATATGCGACCTGGCGCGCACGTTTGCCCGGGAGATGGGGGAGGCTAACTGA
H1-for: 
5'-AAGCTGGGTACCGCGGGCCCGGGATCCACCGGTCGCCACCATG-
GCTTCGTACCCCTGCCATC-3',
H1-rev: 5'-GCCTCGAGCGGCCGCTTCAGTTAGCCTCCCCCATCTC-3'.

The primer pair HSV-TK-for and HSV-TK-rev are used for the qPCR, for titration of HSV-TK-transferring AAV particles:
HSV-TK-for: 5'-GCAGCAAGAAGCCACGGAAG-3',
HSV-TK-rev: 5'-CCAGCAGTTGCGTGGTGGTG-3'.

The primer pairs GFP-for/GFP-rev and luc-for/luc-rev are used for the qPCR, for titration of the vectors:
GFP-for: 5'-GCTACCCCGACCACATGAAG-3',
GFP-rev:5'-GTCCATGCCGAGAGTGATCC-3',
luc-for: 5'-TTCGGCTGGCAGAAGCTATG-3',
luc-rev: 5'-GCTCGCGCTCGTTGTAGATG-3'.

**Vector particle production**. Recombinant Her2-AAV vector particles and AAV-2 were generated using the adenovirus-helper free AAV packaging strategy followed by step gradient centrifugation (Boucas, J. et al., J. Gene Med., 2009, 11, 1103-1113; Anliker, B., et al., Nat. Meth., 2010, 7, 929-935). For production of the recombinant Her2-AAV vector particles, HEK-293 cells were transfected with plasmids pXX6, pRCVP2koA, pDARPin-VP2 and the at least one vector plasmid in an equimolar ratio mixed with polyethylene-imine (PEI) as previously described for lentiviral vectors (Anliker, B., et al., Nat. Meth., 2010, 7, 929-935). For production of AAV-2, which served as control, HEK-293 cells were transfected with plasmids pXX6, pRC (Girod, A. et al., Nat. Meth., 1999, 1052-1056) and the vector plasmid. 48 hours after transfection cells were harvested, pelleted by centrifugation and lysed. Cell lysate was treated by Benzonase (Sigma), pre-cleared by low speed centrifugation and subjected to iodixonal density gradient centrifugation as described previously (Boucas, J. et al., J. Gene Med., 2009, 11, 1103-1113). All vectors contained a self-complementary vector genome conformation. However, vectors can also be packaged with a single-stranded vector genome.

**Determination of genomic and capsid titers**. Genomic particle titers were determined as described previously (Boucas, J. et al., J. Gene Med., 2009, 11, 1103-1113) using primers specific for each transgene (Supplementary Table 1). Capsid titers were determined using an ELISA-based assay. AAV vector preparations were coated on Maxisorp immunoplates (Nunc, Wiesbaden, Germany) and detected with an anti-AAV-2-capsid antibody (A20, 1:4 diluted in PBS containing 3% BSA, 5% sucrose, 0.05% Tween20; Progen, Heidelberg, Germany). Subsequently, plates were incubated with a donkey anti-mouse biotin-conjugated antibody (1:25,000 in PBS containing 3% BSA, 5% sucrose, 0.05% Tween20; Jackson ImmunoResearch, Suffolk, United Kingdom) and HRP-conjugated streptavidin (1:500 in PBS containing 3% BSA, 5% sucrose, 0.05% Tween20; Dianova, Hamburg, Germany). Upon addition of TMB-liquid substrate (Sigma, Hamburg, Germany) according to the manufactures' instruction, the reaction product was quantified at 450 nm wavelength.

**Western Blot**. 2x10¹⁰ genomic particles of iodixanol purified AAV vectors were separated on 8% SDS PAGE and then transferred to nitrocellulose membrane (Amersham Biosciences, Freiburg, Germany). The AAV capsid proteins were detected using the AAV capsid protein specific antibody B1 (Wobus, C. E. et al., J. Virol., 2000, 74, 9281-9293) and secondary anti-mouse peroxidase-conjugated antibodies (Dianova, Hamburg, Germany). Signals were visualized by enhanced chemiluminescense using ECL Plus Western Blotting Detection System (GE Healthcare, Munich, Germany).

**DARPin surface display ELISA.** Serial dilutions of Her2-AAV^{myc} and AAV-2 were bound to ELISA-plates coated with a myc-tag specific antibody (Abcam, Cambridge, United Kingdom). Bound vector particles were quantified using the AAV-2 capsid specific antibody A20 (Progen, Heidelberg, Germany). Subsequent detection was carried out as described under the capsid ELISA section.

**Electron microscopy.** Formvar-carbon coated 300-mesh copper grids were placed on a suspension drop containing iodixanol gradient purified Her2-AA^{myc} or AAV-2 and anti-myc monoclonal antibody (diluted 1:100 in PBS) for 1 hour at room temperature. After washing twice with PBS, the grid was incubated for 30 minutes with 10 nm gold-labeled secondary anti-mouse antibody diluted 1:100 in PBS. Subsequently, grids were washed five times with ddH2O and stained for 15 seconds in 2% aqueous uranyl acetate. Samples were examined in an EM109 transmission electron microscope (Zeiss, Jena, Germany). Representative gold-labeled particles at a magnification of x 140,000 are shown. Scale bar corresponds to 50 nm.

**Competition assay**. AAV-EGFP vector preparations were incubated for one hour at 4°C with increasing amounts of the entire HER2/*neu* receptor extracellular domain (Sino Biological, Beijing, China). Following incubation, SK-OV-3 cells were transduced. After 48 h, the percentage of EGFP-positive cells was determined by flow cytometry.

**Column purification of Her2-AAV. I**odixanol gradient purified Her2-AAV^{His} preparations were further purified through nickel column affinity chromatography. AAV-preparations were loaded to affinity columns at flow rates of 0.1 ml/mn diluted in binding buffer (20 mM imidazole, 20 mM sodium phosphate and 0.5 M NaCl pH 7.4). Columns were washed with 10 column volumes binding buffer, and eluted using step gradient elution (20-500 mM imidazole, 20 mM sodium phosphate and 0.5 M NaCl pH 7.4). Peak fractions were detected using absorbance reading at 280 nm and verified by dot-blot and Western-Blot analysis. Peak fractions were dialyzed.

**In vivo analysis of Her2-AAV**. Experimental mouse work was carried out in compliance with the German animal protection law. For biodistribution analysis, six to eight week old Beige nude mice (Harlan Laboratories, Eystrup, Germany) were engrafted subcutaneously with SK-OV-3 tumor cells as described previously (Münch, R. C. et al., Mol. Ther., 2011, 19, 686-693). AAV-2 and Her2-AAV particles were injected systemically through the tail vein and luciferase signals were detected by *in vivo* imaging one week post injection. For tumor killing experiments, SK-OV-3 cell derived tumor bearing mice received a single intravenous injection of (GOI=8x10¹¹) AAV-HSV-TK or Her2-AAV-HSV-TK, respectively. From two days after vector application on, mice received a daily intraperitoneal injection of GCV (100 mg/kg body weight) for seven consecutive days. Tumor size was determined every three days using calipers.

The level of ALT in blood was determined using the VETTEST-8008-system (IDEXX, Ludwigsburg, Germany) in accordance with the manufacturer's instructions.

For histology, organ or tumor tissue was fixed in 4% formalin for six days, paraffin embedded and 3 µm slices were stained with haematoxylin and eosin.

For *in vivo* imaging, mice were intraperitoneally injected with 150 mg D-Luciferin/kg body weight (Caliper Life Sciences, Mainz, Germany) and anesthetized. Imaging data were obtained 10 minutes after substrate injection using a noninvasive cooled charged-coupled device (IVIS Spectrum; Caliper Life Sciences). Data were analyzed using the Living Image Software (Caliper Life Sciences).

**Statistics**. Data were analyzed using the unpaired two-tailed t-test. Survival curves were analyzed by the Kaplan-Meier log-rank test. P ≤ 0.05 was taken to be significant. Graph Pad Prism 5 provided the software for statistical analysis.

### Example 2: Monitoring of in vitro and in vivo transduction

To monitor in vitro and in vivo transduction, the cDNA for EGFP and firefly luciferase (luc-2) was packaged. All genes had been placed under the control of the strong and ubiquitously active spleen focus forming virus (SSFV) promoter (Baum, C. et al., J. Virol., 1995, 69, 7541-7547). Their efficient packaging into Her2-AAV particles as well as incorporation of the DARPin-VP2 fusion protein was confirmed (Fig.1 b and 1 c). Furthermore, as demonstrated by ELISA and electron microscopy, DARPin molecules were efficiently displayed on the surface of AAV particles and accessible for binding partners (Fig. 1 d and 1 e).

### Example 3: Evaluation of the targeting potential of the recombinant AAV vector particles

To evaluate the targeting potential of DARPin-AAV vector particles, the gene delivery into a panel of target receptor-positive and -negative cell types was assessed. Specifically, the gene delivery into HER2/*neu*-negative Chinese hamster ovarian cells (CHO), the CHO cell line CHO Her2-k6, which stably expresses HER2/*neu*, and into the ovarian cancer cell line SK-OV-3, naturally over-expressing HER2/*neu* (Münch, R. C. et al., Mol. Ther., 2011, 19, 686-693), was assessed. Stocks of Her2-AAV particles reached averages titers of 2 x 10⁸ t.u./ml on HER2/*neu*positive SK-OV-3 cells, which was about 30-fold less than for AAV-2 particles. Her2-AAV particles were also capable of efficiently transducing CHO-Her2-k6 cells, whereas transduction of HER2/*neu*-negative CHO-K1 cells remained at background levels, even when a high vector dose was applied (Fig. 2a). The transduction efficiency of Her2-AAV particles correlated with the HER2/*neu*-receptor density on target cells (Fig. 2c) and was independent of HSPG, as indicated by the intensity to competition with heparin, the soluble analogue of HSPG (Fig. 2a). In contrast, AAV-2-mediated transduction was completely blocked by heparin (Fig. 2a). The HER2/*neu*-specificity of Her2-AAV was further demonstrated by inhibiting transduction with the soluble extracellular domain of the HER2/*neu*-receptor (Fig. 2b). To evaluate the targeting capacity of Her2-AAV particles for highly underrepresented target cells, a series of cell mixtures comprising defined ratios of CHO-Her2-k6 and CHO-K1 cells was transduced. Her2-AAV particles transduced more than 50% of the target cell population even when representing only 5% of the total cell number, further indicating the high level of specificity (Fig. 2c).

### Example 4: Evaluation of the targeting capacity of recombinant AAV vector particle comprising the HER2/neu-specific DARPin

The Luciferase-expression was used to evaluate the targeting capacity of Her2-AAV *in vivo.* To this end, it has been demonstrated that Her2-AAV particles carrying the luc-2 expression cassette were capable of discriminating between HER2/*neu*-positive and -negative cells *in vivo.* The vectors were then injected into the tail vein of nude mice carrying subcutaneous SK-OV-3 tumors. Luciferase expression was regularly analyzed using charged-coupled device imaging. By day seven, Her2-AAV-injected mice showed strong luciferase activity confined on the tumor (Fig. 5a). In sharp contrast, no signal was detectable in the tumors of AAV-2-injected mice, which instead showed prominent signals in the liver (Fig. 5a). Quantification of the luciferase activities in organ lysates confirmed the imaging data. Luciferase expression in the tumor tissue of Her2-AAV-injected animals exceeded the activities elsewhere in the body by at least 20-fold (Fig. 1e). In contrast, AAV-2-injected mice produced the most prominent signal in the liver, while the tumor tissue emitted signals just above background. Luciferase activity, however, depends on transgene expression. In order to assess, if the targeting strategy also re-directed particle distribution to the target tissue, the AAV genome copy numbers in tumor and non-target cells has been quantified. AAV-2 particles accumulated mainly in the liver, which contained more than 100-fold more genome copies than the tumor tissue. All other tested organs contained at least 10-fold more vector particles than the tumor tissue. The distribution of Her2-AAV genome copies was completely different. Here, tumor tissue contained between 10- (kidney) to 100-fold (liver) more genome copies than the other organs (Fig. 5b). Thus, relative to liver, Her2-AAV particles were by more than four orders of magnitude more efficient in targeting the tumor tissue than AAV-2 particles. This extent of targeting tissue and efficiency upon systemic administration is remarkable and has not been described before for any non-enveloped viral vector.

### Example 5: Complete restriction of AAV vector tropism by separation of DARPin-proficient and DARPin-deficient particles

As VP2 is not essential for capsid assembly, it has been hypothesized that Her2-AAV vector preparations consist of DARPin-proficient and -deficient particles. The latter are composed of VP1 and VP3, are deficient in HSPG binding and lack the targeting ligand. This hypothesis is supported by the weaker band of DARPin-VP2 detected for Her2-AAV (and Her2-AAV^{HSPG+}) compared to VP2 for AAV-2 detected by Western Blot (Fig. 1 c). Although non-infectious *in vitro,* AAV vectors deficient in HSPG binding have been reported to transduce heart and to a lower extend lung tissue following intravenous injection. In order to evaluate whether the weak signals observed in the chest region are caused by DARPin-deficient particles in Her2-AAV stocks, AAV-2^{ΔHSPG} in comparison to Her2-AAV preparations have been injected into SK-OV-3 bearing mice. In AAV-2^{ΔHSPG} treated animals luciferase signals were confined to the chest region, whereas Her2-AAV mediated signals again were clearly located at the tumor site (Fig. 6). These findings suggest that the weak off-targeting signals in the chest region observed in mice injected with Her2-AAV are indeed caused by DARPin-deficient particles. To reduce these off-targeting effects, a His-tag was fused to the N-terminal end of the DARPin-VP2 fusion construct and incorporated into Her2-AAV particles (Her2-AAV^{His}). Her2-AA V^{His} particles revealed enhanced binding affinity to nickel based affinity chromatography columns as compared to DARPin-deficient particles displaying no His-tag. Dot-Blot and Western-Blot analysis of elution fractions from His-Trap-HP-columns confirmed that DARPin-deficient particles did not bind to Ni-columns, whereas Her2-AAV^{His} could be eluted in distinct fractions (Fig. 7). These purified Her2-AAV vector particles are able to transduce HER2/*neu*-positive cells, distinguish between HER2/*neu*-positive and -negative cells, and are expected to exclusively transduce tumor tissue upon intravenous injection into tumor bearing mice.

### Example 6: Demonstration of the tumor cell killing by recombinant AAV vector particle comprising the HER2/neu-specific DARPin

So far, lack of specificity has impaired the use of recombinant AAV vector particles for suicide gene induced tumor cell killing. To demonstrate that Her2-AAV particles are suitable for tumor cell killing following systemic vector delivery and significantly contributes to vector safety, Her2-AAV particles have been equipped with the Herpes simplex thymidine kinase (HSV-TK), which converts ganciclovir (GCV) into cytotoxic compounds (Raty, J. K. et al., Curr. Mol. Pharmacol., 2008, 1, 13-23). *In vitro,* this vector - in contrast to AAV-2 - killed HER2/*neu*-positive cell lines in a GCV dose-dependent manner while HER2/*neu*-negative cells remained unaffected (Fig. *8**). In vivo,* a single systemic injection of Her2-AAV particles into tumor-bearing mice was sufficient to significantly delay tumor growth, while the tumor volume of AAV-2-treated animals increased at the same rate as tumors in control animals (Fig. 5c and Fig. 3). Of note, although the tumors of AAV-2-injected mice were still far below the critical size, half of these animals developed liver failure, substantial weight loss and had to be sacrificed within the first days of GCV-treatment (N=6, P=0,05; Logrank test) (Fig. 9a and Fig. 9b) Further investigations of these mice revealed elevated serum transaminase (ALT) levels (Fig. 5d) and massive acute multifocal central lobular necrosis as typically induced by hepatotoxins (Fig. 5e and Fig. 9).

### Example 7: Demonstration of the anti-HIV-treatment by recombinant AAV vector particle comprising the CD4-specific DARPin

Besides tumor-associated antigens, cellular components of the blood are desired targets for gene transfer. Of particular interest for anti-HIV treatment strategies are CD4 T lymphocytes. Therefore, DARPin 55.2 has been chosen, which is specific for human CD4 (Schweizer, A. et al., PLoS Pathog., 2008, 4, e1000109), to further assess the potency of the new targeting platform of the present invention. CD4-AAV particles were cloned and produced as EGFP-expressing vector as described for Her2-AAV particles. Incorporation of DARPin 55.2 as DARPin-VP2 fusion protein was confirmed by Western Blot (data not shown). Next, the target cell specificity of CD4-AAV particles has been assessed in comparison to AAV-2 particles. AAV-2 particles transduced CD4-positive and -negative cells with similar efficiency. In contrast, CD4-AAV particles solely transduced CD4-positive A3.01 T cells (Fig. 10). No EGFP expression was detectable in CD4-negative cell lines. These findings indicate that combining high affinity receptor binding conferred by DARPins with natural receptor ablation completely restricts the tropism of AAV to a receptor of choice.

## Claims

1. Recombinant Adeno-Associated Virus (AAV) vector particle, wherein the differences to the AAV wild-type particle are based on the following features:
(a) at least one packaged transgene,
(b) a mutated AAV capsid protein VP2, wherein the mutation is based on a cell-type specific designed ankyrin repeat proteins (DARPin) that is fused to the N-terminus of the AAV capsid protein VP2 (DARPin-VP2 fusion protein),
(c) a mutated VP2-start codon, and
(d) a capsid, wherein the essential binding site for the natural receptor of the AAV capsid proteins VP1, VP2 and VP3 is mutated.

2. Recombinant AAV vector particle according to claim 1, wherein the at least one transgene of feature (a) comprises a transgene that encodes for a therapeutically active product and/or a transgene that encodes for a marker/reporter protein.

3. Recombinant AAV vector particle according to claim 1 or 2, wherein a removable His-tag is fused to the N-terminal end of the DARPin-VP2 fusion protein.

4. Recombinant AAV vector particle according to any of claims 1 to 3, wherein the cell-type specific DARPin of feature (b) is specific for at least one surface receptor that is expressed on a target cell or target tissue.

5. Recombinant AAV vector particle according to any of claims 1 to 4, wherein the VP2-start codon of feature (c) is mutated by point mutation.

6. Recombinant AAV vector particle according to any of claim 1 to 5, wherein the essential binding site of feature (d) is mutated at at least two amino acid residues in the common VP3 region of each capsid protein.

7. Recombinant AAV vector particle according to claim 6, wherein the R585 und R588 amino acid residues are mutated to alanine.

8. Recombinant AAV vector particle according to any of claims 1 to 7, wherein the DARPin is HER2/*neu*-specific or CD4-specific.

9. Recombinant AAV vector particle according to claim 8, wherein the DARPin is selected from the group consisting of DARPin 9.29 and 55.2.

10. Recombinant AAV vector particle according to claims 1 to 9 for use as a medicament.

11. Recombinant AAV vector particle for use according to claim 10 in the treatment or prevention of at least one condition in a subject, wherein the condition is selected from the group comprising a chronic infection, an inherited monogenetic disease, a cardiovascular disease, a neurodegenerative disease, cancer, HIV, Alzheimer disease, Parkinson disease, diabetes, a neuroinflammatory disease, a rheumatic disease, an autoimmune disease, adipositas, acute lymphoblastic leukemia, myeloid leukemia, renal carcinoma, and disorders related to endothelialization or re-endothelialization, wherein the cancer is selected from the group comprising leukemia, myeloblastic, promyelocytic, myelomonocytic, monocytic, erythroleukemia, chronic myelocytic (granulocytic) leukemia, and chronic lymphocytic leukemia, lymphoma, e.g. Hodgkin's disease and non-Hodgkin's disease, fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilms' tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia, prostate cancer, prostatitis, benign prostatic hypertrophy, benign prostatic hyperplasia (BPH), prostatic paraganglioma, prostate adenocarcinoma, prostatic intraepithelial neoplasia, prostato-rectal fistulas, and atypical prostatic stromal lesions.

12. Recombinant AAV vector particle according to claim 11, wherein the AAV vector particle is administered systemically or *ex vivo.*

13. Method for the construction and generation of the recombinant AAV vector particles according to any of claims 1 to 12, comprising a transfection method, which requires the use of:
(i) a vector plasmid comprising the at least one packaged transgene as described in feature (a),
(ii) a plasmid encoding the DARPin-VP2 fusion protein, wherein VP2 comprises the mutations in the VP2-start codon and in the essential binding site as described in features (c) and (d) of claim 1,
(iii) an AAV helper plasmid encoding for AAV non-structural proteins, preferably viral Rep proteins and AAP, and for the capsid proteins VP1 and VP3, wherein the plasmid comprises the mutations in the VP2-start codon and in the essential binding site as described in features (c) and (d) of claim 1, and
(iv) at least one adenovirus (Ad) helper plasmid encoding for genes possessing helper functions for AAV.

14. The method according to claim 13, wherein the at least one packaged transgene of feature (i) comprises a transgene that encodes for a therapeutically active protein and/or a transgene that encodes for a marker/reporter protein.

15. The method according to claim 13 or 14, wherein a removable His-tag is fused to the N-terminal end of the DARPin-VP2 fusion protein.
